# EUROPEAN PATENT SPECIFICATION

(11) **EP 1 406 922 B1**
(45) Date of publication and mention of the grant of the patent: **09.12.2015**
(21) Application number: 01987457.7
(22) Date of filing: 28.12.2001
(51) Int. Cl.: C07K 14/00, C07K 14/505

(54) **PROTECTION, RESTORATION, AND ENHANCEMENT OF ERYTHROPOIETIN-RESPONSIVE CELLS, TISSUES AND ORGANS**
SCHUTZ, RESTAURATION UND VERSTÄRKUNG VON AUF ERYTHROPOIETIN ANSPRECHENDEN ZELLEN, GEWEBEN UND ORGANEN"
PROTECTION, RESTAURATION ET AMELIORATION DE TISSUS, D'ORGANES ET DE CELLULES SENSIBLES A L'ERYTHROPOIETINE

(30) Priority: 29.12.2000 US 753132; 29.12.2000 US 259245 P
(43) Date of publication of application: 14.04.2004
(60) Divisional application: 10012572.3
(73) Proprietor: The Kenneth S. Warren Institute, Inc., Ossining, NY 10562 (US)
(72) Inventor: BRINES, Michael, Woodbridge, CT 06525 (US); CERAMI, Anthony, New York, NY 10012 (US); CERAMI, Carla, Sleepy Hollow, NY 10591 (US)
(74) Representative: Hoffmann Eitle
(86) International application number: PCT/US2001/049479
(87) International publication number: WO 2002/053580

(56) References cited:
- US-A- 5 625 035
- US-A- 5 767 078
- US-A- 5 955 422

## Description

Priority of provisional application no.60/259,245 filed on December 29,2000, is claimed under 35 U.S.C. § 119(e)(1).

### BACKGROUND OF THE INVENTION

For many years, the only clear physiological role of erythropoietin had been its control of the production of red blood cells. Recently, several lines of evidence suggest that erythropoietin, as a member of the cytokine superfamily, performs other important physiologic functions which are mediated through interaction with the erythropoietin receptor (erythropoietin-R). These actions include mitogenesis, modulation of calcium influx into smooth muscle cells and neural cells, and effects on intermediary metabolism. It is believed that erythropoietin provides compensatory responses that serve to improve hypoxic cellular microenvironment as well as modulate programmed cell death caused by metabolic stress. Although studies have established that erythropoietin injected intracranially protects neurons against hypoxic neuronal injury, intracranial administration is an impractical and unacceptable route of administration for therapeutic use, particularly for normal individuals. Furthermore, previous studies of anemic patients given erythropoietin have concluded that peripherally-administered erythropoietin is not transported into the brain (Marti et al., 1997, Kidney Int. 51:416-8; Juul et al., 1999, Pediatr. Res. 46:543-547; Buemi et al., 2000, Nephrol. Dial. Transplant. 15:422-433.).

Various modified forms of erythropoietin have been described with activities directed towards improving the erythropoietic activity of the molecule, such as those with altered amino acids at the carboxy terminus described in U.S. Patent 5,457,089 and in U.S. Patent 4,835,260; erythropoietin isoforms with various numbers of sialic acid residues per molecule, such as described in U.S. Patent 5,856,292; polypeptides described in U.S. Patent 4,703,008; agonists described in U.S. Patent 5,767,078; peptides which bind to the erythropoietin receptor as described in U.S. Patents 5,773,569 and 5,830,851; and small-molecule mimetics as described in U.S. Patent 5,835,382.

US A 5,955,422 discloses a pharmaceutical composition comprising human EPO. This document does not appreciate that non-erythropoietic EPOs are useful as well.

It is towards the use of a specific erythropoietin for protecting, enhancing, or restoring ve cells and associated cells, tissues and organs *in situ* as well as *ex vivo,* and to delivery of an erythropoietin across an endothelial cell barrier for the purpose of protecting and enhancing erythropoietin-responsive cells and associated cells, tissues and organs distal to the vasculature, or to carry associated molecules, that the present invention is directed.

### BRIEF SUMMARY OF THE INVENTION

In one aspect, the present invention is directed to the use of pharmaceutical composition comprising a modified, non-erythropoietic erythropoietin and a pharmaceutically-acceptable carrier for use in protecting, enhancing or restoring the function or viability of an erythropoietin-responsive neuronal mammalian tissue comprising erythropoietin-responseive cells, wherein said modified erythropoietin has at least one of the following modifications:
- 0, 1, 2, 3, 4, 5, 6, 7, 8, 9, 10, 11, 12, or 13 sialic acid moieties; or
- one or more carbamylated lysine residues and/or a carbamylation of the N-terminal amino group.
In one particular aspect, the erythropoietin-responsive neuronal mammalian cells and their associated cells, tissue or organ are distal to the vasculature by virtue of a tight endothelial cell barrier. In another particular aspect, said cells, tissues, organs or other bodily parts are isolated from a mammalian body, such as those intended for transplant. Said erythropoietin-responsive cell or tissue are neuronal, cells or tissue. In one aspect, said erythropoietin-responsive cell or its associated cells, tissues, or organs are not excitable cells, tissues, or organs. or do not predominantly comprise excitable cells or tissues. In a particular embodiment, said mammalian cell, tissue or organ for which an aforementioned erythropoietin derivative is used are those that have expended or will expend a period of time under at least one condition adverse to the viability of the cell, tissue or organ. Such conditions include traumatic *in-situ* hypoxia or metabolic dysfunction, surgically-induced *in-situ* hypoxia or metabolic dysfunction, or *in-situ* toxin exposure, the latter may be associated with chemotherapy or radiation therapy. In one embodiment, the adverse conditions are a result of cardio-pulmonary bypass (heart-lung machine), as is used for certain surgical procedures.

The erythropoietins as dedined in the claims are useful for the therapeutic or prophylactic treatment of human diseases of the CNS or peripheral nervous system which have primarily neurological or psychiatric symptoms, as well as ophthalmic diseases, cardiovascular diseases, cardiopulmonary diseases, respiratory diseases, kidney, urinary and reproductive diseases, gastrointestinal diseases and endocrine and metabolic abnormalities, as long as those involve the neuronal tissue as defined in the claims.

The invention is also directed to the use pharmaceutical compositions comprising particular erythropoietin derivatives as defined in the claims for administration to a mammalian animal, preferably a human. Such pharmaceutical compositions may be formulated for oral, intranasal, or parenteral administration, or in the form of a perfusate solution for maintaining the viability of cells, tissues or organs *ex vivo.*

Erythropoietin derivatives useful for the aforementioned purposes and pharmaceutical compositions include both native erythropoietins as well as erythropoietins that have been altered by at least one modification as compared to native erythropoietin, and preferably as compared to native human erythropoietin. The at least one modification is a modification as defined in claim 1. Of course, erythropoietin molecules useful for the purposes herein may have a plurality of modifications compared to the native molecule, such as multiple modifications of the amino acid portion of the molecule, multiple modifications of the carbohydrate portion of the molecule, or at least one modification of the amino acid portion of the molecule and at least one modification of the carbohydrate portion of the molecule. The modified erythropoietin molecule retains its ability of protecting, enhancing or restoring the function or viability of erythropoietin-responsive neuronal mammalian cells, yet other properties of the erythropoietin molecule unrelated to the aforementioned, desirable feature may be absent as compared to the native molecule. The erythropoietin derivative is non-erythropoietic.

In one embodiment, the erythropoietin of the invention has at least no sialic acid moieties. In a preferred embodiment, the modified erythropoietin is asialoerythropoietin, and most preferably, human asialoerythropoietin. In another embodiment, the modified erythropoietin has 1, 2, 3, 4, 5, 6, 7, 8, 9, 10, 11, 12, or 13 sialic acid moieties.

In a second embodiment, the modified erythropoietin has at least no N-linked or no O-linked carbohydrates.

In a third embodiment, the modified erythropoietin has at least a reduced carbohydrate content by virtue of treatment of erythropoietin with its native carbohydrates with at least one glycosidase.

In a fourth embodiment, the carbohydrate portion of the modified erythropoietin molecule has at least a non-mammalian glycosylation pattern by virtue of the expression of a recombinant erythropoietin in non-mammalian cells. In preferred embodiments, the modified erythropoietins are expressed in insect cells or plant cells.

In a fifth embodiment, the modified erythropoietin has at least one or more oxidized carbohydrates which also may be chemically reduced. In a preferred embodiment, the modified erythropoietin is periodate-oxidized erythropoietin; in another preferred embodiment, the periodate-oxidized erythropoietin is chemically reduced with a borohydride salt such as sodium borohydride or sodium cyanoborohydride.

In a sixth embodiment, the modified erythropoietin for the aforementioned uses has at least one or more modified arginine residues. In one embodiment, the modified erythropoietin comprises a glyoxal moiety on the one or more arginine residues, such as an arylglyoxal or alkylglyoxal moiety. In another embodiment, at least one arginine residue is modified by reaction with a vicinal diketone such as but not limited to 2,3-butanedione or cyclohexanedione.

In an embodiment of claim 1, the modified erythropoietin comprises at least one or more modified lysine residues or a modification of the N-terminal amino group of the erythropoietin molecule, such modifications as those resulting from reaction of the lysine residue or N-terminal amino group with an amino-group-modifying agent. The modified lysine residue further may be chemically reduced. In one preferred embodiment, an erythropoietin is biotinylated or carbamylated via one or more lysine groups. In another preferred embodiment, the lysine is reacted with an aldehyde or reducing sugar to form an imine, which may be stabilized by reduction as with sodium cyanoborohydride to form an N-alkylated lysine such as glucitolyl lysine, or which in the case of reducing sugars may be stabilized by Amadori or Heyns rearrangement to form an alpha-deoxy alpha-amino sugar such as alpha-deoxy-alpha-fructosyllysine. In another preferred embodiment, the lysine group is carbamylated (carbamoylated), such as by virtue of reaction with cyanate ion, alkyl-carbamylated, aryl-carbamylated, or aryl-thiocarbamylated with an alkyl-isocyanate, aryl-isocyanate, or aryl-isothiocyanate, respectively, or it may be acylated by a reactive alkylcarboxylic or arylcarboxylic acid derivative, such as by reaction with acetic anhydride, succinic anhydride or phthalic anhydride. At least one lysine group may also be trinitrophenyl modified by reaction with a trinitrobenzenesulfonic acid, or preferably its salts. In another embodiment, lysine residues may be modified by reaction with a glyoxal derivative, such as reaction with glyoxal, methylglyoxal or 3-deoxyglucosone to form the corresponding alpha-carboxyalkyl derivatives.

In an eighth embodiment, at least one tyrosine residue of erythropoietin may be modified in an aromatic ring position by an electrophilic reagent, such as by nitration or iodination.

In a ninth embodiment, at least an aspartic acid or a glutamic acid residue of an erythropoietin may be modified, such as by reaction with a carbodiimide followed by reaction with an amine such as but not limited to glycinamide.

In a tenth embodiment, at least a tryptophan residue of an erythropoietin is modified, such as by reaction with n-bromosuccinimide or n-chlorosuccinimide.

In an eleventh embodiment, a modified erythropoietin molecule is provided having at least one erythropoietin amino group removed, such as by reaction with ninhydrin followed by reduction of the resulting carbonyl group by reaction with borohydride.

In a twelfth embodiment, a modified erythropoietin is provided having at least an opening of at least one of the cystine linkages in the erythropoietin molecule by reaction with a reducing agent such as dithiothreitol, followed by reaction of the subsequent sulfhydryls with iodoacetamide, iodoacetic acid or another electrophile to prevent reformation of the disulfide linkages.

In a thirteenth embodiment, a modified erythropoietin is provided having at least one substitution of any one of a number of amino acids, such as a leucine, with at least one of lysine, arginine, tryptophan, tyrosine, or cysteine residues of erythropoietin, using molecular biological techniques.

In a fourteenth embodiment, a modified erythropoietin is subjected to a limited chemical proteolysis that targets specific residues, for example, to cleave after tryptophan residues. Such resulting erythropoietin fragments are embraced herein.

As noted above, an erythropoietin useful for the purposes herein may have at least one of the aforementioned modifications as defined in claim 1, but may have more than one of the above modifications. By way of example of a modified erythropoietin with one modification to the carbohydrate portion of the molecule and one modification to the amino acid portion, a modified erythropoietin is asialoerythropoietin and has its lysine residues biotinylated or carbamylated. The present invention also embraces compositions, including pharmaceutical compositions, comprising one or more of the aforementioned erythropoietins.

In another aspect of the invention, a method is provided for the protecting, enhancing or restoring the function or viability of erythropoietin-responsive neuronal mammalian cells and their associated cells, tissues and organs, by administering an effective amount of any one or more of the aforementioned erythropoietins. In one particular aspect of the method, the erythropoietin-responsive mammalian cells and their associated cells, tissue or organ are distal to the vasculature by virtue of a tight endothelial cell barrier. In another particular aspect, the cells, tissues, organs or other bodily parts are isolated from a mammalian body, such as those intended for transplant, the erythropoietin-responsive cell or tissue are neuronal cells or tissue. In a particular embodiment, the erythropoietin-responsive cell or its associated cells, tissues, or organs are not excitable cells, tissues, or organs, or do not predominantly comprise excitable cells or tissues. In another particular embodiment, the mammalian cell, tissue or organ for which an aforementioned erythropoietin derivative may be administered are those that have expended or will expend a period of time under at least one condition adverse to the viability of the cell, tissue or organ. Such conditions may include traumatic *in-situ* hypoxia or metabolic dysfunction, surgically-induced *in-situ* hypoxia or metabolic dysfunction, or *in-situ* toxin exposure, the latter may be associated with chemotherapy or radiation therapy. In one embodiment, the invention protects against the adverse conditions resulting from cardio-pulmonary bypass.

In another aspect of the invention, any of the foregoing erythropoietins as well as any other erythropoietin molecules including native human erythropoietin can be used in the preparation of a pharmaceutical composition for *ex-vivo* treatment of cells, tissues and organs for the purpose of protecting, enhancing, or restoring the function or viability of erythropoietin-responsive neuronal mammalian cells and their associated cells, tissues and organs. Such *ex-vivo* treatment is useful, for example, for the preservation of cells, tissues or organs for transplant, whether autotransplant or xenotransplant. The cells, tissue or organ may be bathed in a solution comprising erythropoietin, or the perfusate instilled into the organ through the vasculature or other means, to maintain cellular functioning during the period wherein the cells, tissue or organ is not integrated with the vasculature of the donor or recipient. Administration of the perfusate may be made to a donor prior to organ harvesting, as well as to the harvested organ and to the recipient. Moreover, the aforementioned use of any erythropoietin is useful whenever a cell, tissue or organ is isolated from the vasculature of the individual and thus essentially existing ex *vivo* for a period of time, the term isolated referring to restricting or clamping the vasculature of or to the cell, tissue, organ or bodily part, such as may be performed during surgery, including, in particular, cardio-pulmonary bypass surgery; bypassing the vasculature of the cell, tissue, organ or bodily part; removing the cell, tissue, organ or bodily part from the mammalian body, such may be done in advance of xenotransplantation or prior to and during autotransplantation; or traumatic amputation of a cell, tissue, organ or bodily part. Thus, this aspect of the invention pertains both to the perfusion with an erythropoietin *in situ* and *ex vivo. Ex vivo,* the erythropoietin may be provided in a cell, tissue or organ preservation solution. For either aspect, the exposing may be by way of continuous perfusion, pulsatile perfusion, infusion, bathing, injection, or catheterization.

In yet a further aspect, the invention is directed to a method for protecting, enhancing, or restoring the viability or a neuronal mammalian cell, tissue, organ or bodily part which includes an erythropoietin-responsive cell or tissue, in which the cell, tissue, organ or bodily part is isolated from the mammalian body. The method includes at least exposing the isolated mammalian cell, tissue, organ or bodily part to an amount of an erythropoietin for a duration which is effective to protect, enhance, or restore the aforesaid viability. In non-limiting examples, isolated refers to restricting or clampirig the vasculature of or to the cell, tissue, organ or bodily part, such as may be performed during surgery, in particular, cardio-pulmonary bypass surgery; bypassing the vasculature of the cell, tissue, organ or bodily part; removing the cell, tissue, organ or bodily part from the mammalian body, such may be done in advance of xenotransplantation or prior to and during autotransplantation; or traumatic amputation of a cell, tissue, organ or bodily part. Thus, this aspect of the invention pertains both to the perfusion with an erythropoietin *in situ* and *ex vivo. Ex vivo,* the erythropoietin may be provided in a cell, tissue or organ preservation solution. For either aspect, the exposing may be by way of continuous perfusion, pulsatile perfusion, infusion, bathing, injection, or catheterization.

In the aforementioned isolation or *ex-vivo* embodiment. a useful erythropoietin may be any of the aforementioned erythropoietins, including any modified, non-erthropoietic native erythropoietin, or an erythropoietin analog, an erythropoietin mimetic, and erythropoietin fragment, a hybrid erythropoietin molecule, an erythropoietin-receptor-binding molecule, an erythropoietin agonist, a renal erythropoietin, a brain erythropoietin, an oligomer thereof, a multimer thereof, a mutein thereof, a congener thereof, a naturally-occurring form thereof, a synthetic form thereof, a recombinant form thereof, a glycosylation variant thereof, a deglycosylated variant thereof, or a combination thereof which is modified as defined in the claims. Other erythropoietins include, but are not limited to asialoerythropoietin, N-deglycosylated erythropoietin, O-deglycosylated erythropoietin, erythropoietin with reduced carbohydrate content, erythropoietin with altered glycosylation patterns, erythropoietin with carbohydrates oxidized then reduced, arylglyoxal-inodified erythropoietin, alkylglyoxal-modified erythropoietin, 2,3-butanedione-modified erythropoietin, cyclohexanedione-modified erythropoietin, biotinylated erythropoietin, N-alkylated-lysyl-erythropoietin, glucitolyl lysine erythropoietin, alpha-deoxy-alpha-fructosyllysine-erythropoietin, carbamylated erythropoietin, acetylated erythropoietin, succinylated erythropoietin, alpha-carboxyalkyl erythropoietin, nitrated erythropoietin, iodinated erythropoietin, to name some representative yet non-limiting examples based on the teachings herein. A modified human erythropoietin is preferred . In another embodiment human asialoerythropoietin is preferred. In another embodiment human phenylglyoxal erythropoietin is preferred.

By way of non-limiting examples, the aforementioned *ex-vivo* erythropoietin-responsive cell or tissue is neuronal, cells or tissue.

All of the foregoing methods and uses are preferably applicable to human beings, but are useful as well for any mammal, such as but not limited to companion animals, domesticated animals, livestock and zoo animals. Routes of administration of the aforementioned pharmaceutical compositions include oral, intravenous, intranasal, topical, intraluminal, inhalation or parenteral administration, the latter including intravenous, intraarterial, subcutaneous, intramuscular, intraperitoneal, submucosal or intradermal. For *ex-vivo* use, a perfusate or bath solution is preferred. This includes pervusing an isolated portion of the vasculature *in situ.*

In yet another aspect of the invention, any of the aforementioned erythropoietins are useful in preparing a pharmaceutical composition for restoring a dysfunctional cell, tissue or organ when administered after the onset of the disease or condition responsible for the dysfunction. By way of non-limiting example, administration of a pharmaceutical composition comprising erythropoietin restores cognitive function in animals previously having brain trauma, even when administered long after (e.g., three days, five days, a week, a month, or longer) the trauma has subsided. Erythropoietins useful for such applications include any of the particular aforementioned erythropoietins. A modified human erythropoietin is preferred. In another embodiment human asialoerythropoietin is preferred.

In yet another embodiment, the invention provides methods for the use of the aforementioned erythropoietin for restoring a dysfunctional neuronal cell, tissue or organ when administered after the onset of the disease or condition responsible for the dysfunction. By way of non-limiting example, methods for administration of a pharmaceutical composition comprising erythropoietin restores cognitive function in animals previously having brain trauma, even when administered long after (*e.g.,* three days, five days, a week, a month, or longer) the trauma has subsided. Erythropoietins useful for such methods include any of the particular aforementioned erythropoietins.

A modified human erythropoietin is preferred . In another embodiment human asialoerythropoietin is preferred.

These and other aspects of the present invention will be better appreciated by reference to the following Figures and Detailed Description.

### BRIEF DESCRIPTION OF THE FIGURES

Figure 1 depicts the translocation of parenterally-administered erythropoietin into the cerebrospinal fluid.
Figure 2 shows the protection of the myocardium from ischemic damage by erythropoietin after temporary vascular occlusion.
Figure 3 shows the maintenance of the function of a heart prepared for transplantation by erythropoietin.
Figure 4 compares the *in-vitro* efficacy of erythropoietin and asialoerythropoietin on the viability of serum-starved P19 cells.
Figure 5 is another experiment which compares the *in-vitro* efficacy of erythropoietin and asialoerythropoietin on the viability of serum-starved P19 cells.
Figure 6 compares the *in-vitro* efficacy of erythropoietin and phenylglyoxal-modified erythropoietin on the viability of serum-starved P19 cells.
Figure 7 shows protection of erythropoietin and asialoerythropoietin in a rat focal cerebral ischemia model.
Figure 8 shows a dose response comparing the efficacy of human erythropoietin and human asialoerythropoietin in middle cerebral artery occlusion in a model of ischemic stroke
Figure 9 shows the effect of biotinylated erythropoietin and asialoerythropoietin in the P19 assay.
Figure 10 shows the activity of iodinated erythropoietin in the P 19 assay.
Figure 11 depicts the effects of erythropoietin treatment in a rat glaucoma model.
Figure 12 shows the extent of preservation of retinal function by erythropoietin in the rat glaucoma model.
Figure 13 depicts the restoration of cognitive function following brain trauma by administration of erythropoietin starting five days after trauma.
Figure 14 depicts the restoration of cognitive function following brain trauma by administration of erythropoietin starting 30 days after trauma.
Figure 15 depicts the efficacy of human asialoerythropoietin in a kainate model of cerebral toxicity.

### DETAILED DESCRIPTION OF THE INVENTION

"Erythropoietin-responsive cell" refers to a mammalian cell whose function or viability may be maintained, promoted, enhanced, regenerated, or in any other way benefitted, by exposure to an erythropoietin. Non-limiting examples of such cells include neuronal, retinal, muscle, heart, lung, liver, kidney, small intestine, adrenal cortex, adrenal medulla, capillary endothelial, testes, ovary, and endometrial cells. Moreover, such erythropoietin-responsive cells and the benefits provided thereto by an erythropoietin may be extended to provide protection or enhancement indirectly to other cells that are not directly erythropoietin responsive, or of tissues or organs which contain such non-erythropoietin-responsive cells. These other cells, or tissues or organs which benefit indirectly from the enhancement of erythropoietin-responsive cells present as part of the cells, tissue or organ as "associated" cells, tissues and organs. Thus, benefits of an erythropoietin as described herein may be provided as a result of the presence of a small number or proportion of erythropoietin-responsive cells in a tissue or organ, for example, excitable or neuronal tissue present in such tissue, or the Leydig cells of the testis, which makes testosterone. In one aspect, the erythropoietin-responsive cell or its associated cells, tissues, or organs are not excitable cells, tissues, or organs, or do not predominantly comprise excitable cells or tissues.

The methods of the invention provide for the local or systemic protection or enhancement of cells, tissues and organs within a mammalian body, under a wide variety of normal and adverse conditions, or protection of those which are destined for relocation to another mammalian body. In addition, restoration or regeneration of dysfunction is also provided. As mentioned above, the ability of an erythropoietin to cross a tight endothelial cell barrier and exert its positive effects on erythropoietin-responsive cells (as well as other types of cells) distal to the vasculature offers the potential to prevent as well as treat a wide variety of conditions and diseases which otherwise cause significant cellular and tissue damage in an animal, including human, and moreover, permit success of heretofore unattemptable surgical procedures for which risk traditionally outweighed the benefits. The duration and degree of purposeful adverse conditions induced for ultimate benefit, such as high-dose chemotherapy, radiation therapy, prolonged *ex-vivo* transplant survival, and prolonged periods of surgically-induced ischemia, may be carried out by taking advantage of the invention herein. However, the invention is not so limited, but includes as one aspect, methods or compositions wherein the target erythropoietin-responsive cells are distal to the vasculature by virtue of an endothelial-cell barrier or endothelial tight junctions. In general, the invention is directed to any erythropoietin-responsive cells and associated cells, tissues and organs which may benefit from exposure to an erythropoietin. Furthermore, cellular, tissue or organ dysfunction may be restored or regenerated after an acute adverse event (such as trauma) by exposure to an erythropoietin.

The invention is therefore directed generally to the use of the modified erythropoietins for the preparation of pharmaceutical compositions for the aforementioned purposes in which cellular function is promoted, enhanced, regenerated, or in any other way benefitted. The invention is also directed to methods for enhancing, promoting, or regenerating cellular function by administering to a mammal an effective amount of an erythropoietin as described herein. The invention is further directed to methods for promoting, enhancing, or regenerating cellular function *ex vivo* by exposing cells, a tissue or organ to an erythropoietin. The invention is also directed to a perfusate composition comprising a modified erythropoietin for use in organ or tissue preservation.

The various methods of the invention utilize a pharmaceutical composition which at least includes said erythropoietin at an effective amount for the particular route and duration of exposure to exert positive effects or benefits on erythropoietin-responsive cells within or removed from a mammalian body. Where the target cell, tissues or organs of the intended therapy require the erythropoietin to cross an endothelial cell barrier, the pharmaceutical composition includes the erythropoietin at a concentration which is capable, after crossing the endothelial cell barrier, of exerting its desirable effects upon the erythropoietin-responsive cells. Molecules capable of interacting with the erythropoietin receptor and modulating the activity of the receptor, herein referred to as erythropoietin or erythropoietin receptor activity modulators, are useful in the context of the present invention. These molecules may be, for example, naturally-occurring, synthetic, or recombinant forms of erythropoietin molecules, as described above, or other molecules which may not necessarily resemble erythropoietin in any manner, except to modulate erythropoietin responsive cell activity, as described herein.

Erythropoietin is a glycoprotein hormone which in humans has a molecular weight of about 34 kDa. The mature protein comprises 165 amino acids, and the glycosyl residues comprise about 40% of the weight of the molecule. The forms of erythropoietin useful in the practice of the present invention encompass the above defined modified erythropoietins.

In addition, erythropoietin forms useful in the practice of the present invention include proteins that represent functionally equivalent gene products. Such an equivalent erythropoietin gene product include mutant erythropoietins, which may contain deletions, including internal deletions, additions, including additions yielding fusion proteins, or conservative substitutions of amino acid residues within and/or adjacent to the amino acid sequence, but that result in a "silent" change, in that the change produces a functionally equivalent erythropoietin. Such amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid. Alternatively, non-conservative amino acid changes, and larger insertions and deletions may be used to create functionally altered erythropoietin mutants. Such mutants can be used to alter erythropoietin properties in desirable ways. For example, in one embodiment, an erythropoietin useful for the practice of the invention can be a mutant erythropoietin altered in one or more amino acids within the four functional domains of erythropoietin which affect receptor binding: VLQRY and/or TKVNFYAW and/or SGLRSLTTL and/or SNFLRG. In another embodiment, erythropoietins containing mutations in the surrounding areas of the molecule which affect the kinetics or receptor-binding properties of the molecule can be used.

The term "erythropoietin" as well as "an erythropoietin" may be used interchangeably or conjunctively, and the various analogs, fragments, hybrid molecules, agonists, muteins, and other forms as described above embrace the variants in the extents of and sites of glycosylation of erythropoietin, including native, deglycosylated, asialylated, and other partially glycosylated forms of erythropoietin. Non-limiting examples of such variants are described in Tsuda et al., 1990, Eur. J. Biochem. 188:405-411, incorporated herein by reference. Bacteria, yeast, insect, plant, mammallian, including human. In addition, a variety of host systems may be used for expression and production of recombinant erythropoietin, including, but not limited to, bacteria, yeast, insect, plant, and mammalian, including human, cell systems. For example, recombinant erythropoietin produced in bacteria, which do not glycosylate or sialate the product, could be used to produce non-glycosylated forms of erythropoietin. Alternatively, recombinant erythropoietin can produced in other systems that do glycosylate, e.g., plants, including human cells.

As noted above, the invention herein embraces any and all erythropoietin receptor activity modulator molecules capable of exerting positive activity on erythropoietin-responsive cells, regardless of any structural relationship of the molecule with erythropoietin.

In addition, erythropoietin itself may be modified to tailor its activities for a specific tissue or tissues. Several non-limiting strategies which may be carried out to achieve this desired tissue specificity include modifications that shorten circulating half-life and thus reducing the time erythropoietin can interact with erythroid precursors, or modification of the primary structure of the erythropoietin molecule. One approach to reducing circulating half life is to remove or modify the glycosylation moieties, of which erythropoietin has three N-linked and one O-linked. Such variants of glycosylated erythropoietin can be produced in a number of ways. For example, the sialic acids which terminate the end of the sugar chains can be removed by specific sialidases depending on the chemical linkage connecting the sialic acid to the sugar chain. Alternatively, the glycosylated structure can be dismantled in different ways by using other enzymes that cleave at specific linkages. Techniques to modify the primary structure are myriad and include substitution of specific amino acids, chemical modification of amino acids, or addition of other structures which interfere with the interaction of erythropoietin with any of its receptors. Use of such forms of erythropoietin are fully embraced herein. In a preferred embodiment, the half-life of the non-erythropoietic erythropoietin of the invention is reduced by about 90% from that of native erythropoietin.

Some of these molecules will nevertheless mimic the actions of erythropoietin itself in other tissues or organs. For example, a 17-mer containing the amino-acid sequence of 31-47 of native erythropoietin is inactive for erythropoiesis but fully active for neural cells *in vitro* (Campana & O'Brien, 1998: Int. J. Mol. Med. 1:235-41).

Furthermore, derivative erythropoietin molecules desirable for the uses described herein may be generated by guanidination, amidination, carbamylation (carbamoylation), trinitrophenylation, acetylation, succinylation, nitration, or modification of arginine, lysine, tyrosine, tryptophan, or cysteine residues or carboxyl groups, among other procedures, such as limited proteolysis, removal of amino groups, and/or mutational substitution of arginine, lysine, tyrosine, tryptophan, or cysteine residues by molecular biological techniques to produce erythropoietins which maintain an adequate level of activities for specific organs and tissues but not for others, such as erythrocytes (e.g., Satake et al; 199.0, Biochim. Biophys. Acta 1038:125-9; incorporated herein by reference in its entirety). One non-limiting example as described hereinbelow is the modification of erythropoietin arginine residues by reaction with a glyoxal such as phenylglyoxal (according to the protocol of Takahashi, 1977, J. Biochem. 81:395-402). As will be seen below, such an erythropoietin molecule fully retains its neurotrophic effect. Such erythropoietin molecules are fully embraced for the various uses and compositions described herein.

Synthetic and recombinant molecules, such as brain erythropoietin and renal erythropoietin, recombinant mammalian forms of erythropoietin, as well as its naturally-occurring, tumor-derived, and recombinant isoforms, such as recombinantly-expressed molecules and those prepared by homologous recombination are provided herein. Furthermore, the present invention includes molecules including peptides which bind the erythropoietin receptor, as well as recombinant constructs or other molecules which possess part or all of the structural and/or biological properties of erythropoietin, including fragments and multimers of erythropoietin or its fragments. Erythropoietin herein embraces molecules with altered erythropoietin receptor binding activities, preferably with increased receptor affinity, in particular as pertains to enhancing transport across endothelial cell barriers. Muteins comprising molecules which have additional or reduced numbers of glycosylation sites are included herein. As noted above, the terms "erythropoietin" and "mimetics" as well as the other terms are used interchangeably herein to refer to the erythropoietin-responsive cell protective and enhancing molecules related to erythropoietin as well as the molecules which are capable of crossing endothelial cell barriers. Furthermore, molecules produced by transgenic animals are also encompassed here. It should be noted that erythropoietin molecules as embraced herein do not necessarily resemble erythropoietin structurally or in any other manner, except for ability to interact with the erythropoietin receptor or modulate erythropoietin receptor activity or activate erythropoietin-activated signaling cascades, as described herein.

By way of non-limiting examples, forms of erythropoietin useful for the practice of the present invention include erythropoietin muteins, such as those with altered amino acids at the carboxy terminus described in U.S. Patent 5,457,089 and in U.S. Patent 4,835,260; asialoerythropoietin and erythropoietin isoforms with various numbers of sialic acid residues per molecule, such as described in U.S. Patent 5,856,298; polypeptides described in U.S. Patent 4,703,008; agonists described in U.S. Patent 5,767,078; peptides which bind to the erythropoietin receptor as described in U.S. Patents 5,773,569 and 5,830,851; small-molecule mimetics which activate the erythropoietin receptor, as described in U.S. Patent 5,835,382; and erythropoietin analogs described in WO 9505465, WO 9718318, and WO 9818926. All of the aforementioned citations are incorporated herein to the extent that such disclosures refer to the various alternate forms or processes for preparing such forms of the erythropoietins of the present invention.

Erythropoietin can be obtained commercially, for example, under the trademarks of PROCRIT, available from Ortho Biotech Inc., Raritan, NJ, and EPOGEN, available from Amgen, Inc., Thousand Oaks, CA.

The activity (in units) of erythropoietin (erythropoietin) and erythropoietin-like molecules is traditionally defined based on its effectiveness in stimulating red cell production in rodent models (and as derived by international standards of erythropoietin). One unit (U) of regular erythropoietin (MW of ∼ 34,000) is ∼ 8 ng of protein (1 mg protein is approximately 125,000 U). However, as the effect on erythropoiesis is incidental to the desired activities herein and may not necessarily be a detectable property of certain of the erythropoietins of the invention, the definition of activity based on erythropoietic activity is inappropriate. Thus, as used herein, the activity unit of erythropoietin or erythropoietin-related molecules is defined as the amount of protein required to elicit the same activity in neural or other erythropoietin-responsive cellular systems as is elicited by WHO international standard erythropoietin in the same system. The skilled artisan will readily determine the units of a non-erythropoietic erythropoietin or related molecule following the guidance herein.

Further to the above-mentioned erythropoietin modifications useful herein, the following discussion expands on the various erythropoietins of the invention.

An erythropoietin of the invention may have at least no sialic acid moieties, referred to as asialoerythropoietin. Preferably, an erythropoietin of the invention is human asialoerythropoietin. In alternative embodiments, the erythropoietin of the invention may have at least 1, 2, 3, 4, 5, 6, 7, 8, 9, 10, 11, 12, or 13 sialic acid residues. It may be prepared by desialylating erythropoietin using a sialidase, such as is described in the manufacturer's packaging for Sialydase A from ProZyme Inc., San Leandro, California. Typically, PROZYME® GLYCOPRO® sequencing-grade SIALYDASE A™ (N-acetylneuraminate glycohydrolase, EC 3.2.1.18) is used to cleave all non-reducing terminal sialic acid residues from complex carbohydrates and glycoproteins such as erythropoietin. It will also cleave branched sialic acids (linked to an internal residue). Sialydase A is isolated from a clone of *Arthrobacter ureafaciens.*

### Additionally to the modifications of claim 1, there can be the following modifications:

An erythropoietin may have at least a reduced number of N-linked carbohydrates. To remove N-linked carbohydrates, erythropoietin may be treated with hydrazine, in accordance, for example, with the methods described by Hermentin et al., 1996, Glycobiology 6(2):217-30. As noted above, erythropoietin has three N-linked carbohydrate moieties; the present invention embraces those erythropoietins with two, one, or no N-linked carbohydrate.

An erythropoietin of the invention may have at least a reduced carbohydrate content by virtue of treatment of native erythropoietin with at least one glycosidase. For example, the procedure of Chen and Evangelista, 1998, Electrophoresis 19(15):2639-44, may be followed. Furthermore, removal of the O-linked carbohydrate may be achieved following the methods described in Hokke et al., 1995, Eur. J. Biochem.228(3):981-1008.

The carbohydrate portion of an erythropoietin molecule may have at least a non-mammalian glycosylation pattern by virtue of the expression of a recombinant erythropoietin in non-mammalian cells. Preferably, the erythropoietins are expressed in insect or plant cells. By way of non-limiting example, expression of erythropoietin in insect cells using a baculovirus expression system may be carried out in accordance with Quelle et al., 1989, Blood 74(2):652-657. Another method is described in U.S. Patent 5,637,477. Expression in a plant system may be carried out in accordance with the method of Matsumoto et al., 1993, Biosci. Biotech. Biochem. 57(8):1249-1252. Alternatively, expression in bacteria will result in non-glycosylated forms of erythropoietin. These are merely exemplary of methods useful for the production of an erythropoietin of the invention are in no way limiting.

An erythropoietin of the invention may have at least one or more oxidized carbohydrates that also may be chemically reduced. For example, the erythropoietin may be periodate-oxidized erythropoietin; the periodate-oxidized erythropoietin also may be chemically reduced with a borohydride salt such as sodium borohydride or sodium cyanoborohydride. Periodate oxidation of erythropoietin may be carried out, for example, by the methods described by Linsley et al., 1994, Anal. Biochem.219(2):207-17. Chemical reduction following periodate oxidation may be carried out following the methods of Tonelli and Meints, 1978, J. Supramol. Struct. 8(1):67-78.

An erythropoietin for the aforementioned uses may have at least one or more modified arginine residues. For example, the modified erythropoietin may comprise a R-glyoxal moiety on the one or more arginine residues, where R may be an aryl, heteroaryl, lower alkyl, lower alkoxy, or cycloalkyl group, or an alpha-deoxyglycitolyl group. As used herein, the term lower "alkyl" means a straight- or branched-chain saturated aliphatic hydrocarbon group preferably containing 1-6 carbon atoms. Representative of such groups are methyl, ethyl, isopropyl, isobutyl, butyl, pentyl, hexyl and the like. The term "alkoxy" means a lower alkyl group as defined above attached to the remainder of the molecule by oxygen. Examples of alkoxy include methoxy, ethoxy, propoxy, isopropoxy and the like. The term "cycloalkyl" refers to cyclic alkyl groups with three up to about 8 carbons, including for example cyclopropyl, cyclobutyl, cyclohexyl and the like. The term aryl refers to phenyl and naphthyl groups. The term heteroaryl refers to heterocyclic groups containing 4-10 ring members and 1-3 heteroatoms selected from the group consisting of oxygen, nitrogen and sulfur. Examples include but are not limited to isoxazolyl, phenylisoxazolyl, furyl, pyrimidinyl, quinolyl, tetrahydroquinolyl, pyridyl, imidazolyl, pyrrolidinyl, 1,2,4-triazoylyl, thiazolyl, thienyl, and the like. The R group may be substituted, as for example the 2,3,4-trihydroxybutyl group of 3-deoxyglucosone. Typical examples of R-glyoxal compounds are glyoxal, methylglyoxal, 3-deoxyglucosone, and phenylglyoxal. Preferred R-glyoxal compounds are methylglyoxal or phenylglyoxal. An exemplary method for such modification may be found in Werber et al., 1975, Isr. J. Med. Sci. 11(11): 1169-70, using phenylglyoxal.

In a further example, at least one arginine residue may be modified by reaction with a vicinal diketone such as 2,3-butanedione or cyclohexanedione, preferably in ca. 50 millimolar borate buffer at pH 8-9. A procedure for the latter modification with 2,3-butanedione may be carried out in accordance with Riordan, 1973, Biochemistry 12(20): 3915-3923; and that with cyclohexanone according to Patthy et al., 1975, J. Biol. Chem 250(2): 565-9.

An erythropoietin of the invention may comprise at least one or more modified lysine residues or a modification of the N-terminal amino group of the erythropoietin molecule, such modifications as those resulting from reaction of the lysine residue with an amino-group-modifying agent. In another embodiment, lysine residues may be modified by reaction with glyoxal derivatives, such as reaction with glyoxal, methylglyoxal and 3-deoxyglucosone to form alpha-carboxyalkyl derivatives. Examples are reaction with glyoxal to form carboxymethyllysine as in Glomb and Monnier, 1995, J. Biol. Chem. 270(17):10017-26, or with methylglyoxal to form (1-carboxyethyl)lysine as in Degenhardt et al., 1998, Cell. Mol. Biol. (Noisy-le-grand) 44(7):1139-45. The modified lysine residue further may be chemically reduced. For example, the erythropoietin may be biotinylated via lysine groups, such as in accordance with the method described in Example 5, in which D-biotinoyl-ε-aminocaproic acid-N-hydroxysuccinimide ester was reacted with erythropoietin, followed by removal of unreacted biotin by gel filtration on a Centricon 10 column, as described by Wojchowski and Caslake, 1989, Blood 74(3):952-8. In this paper, the authors use three different methods of biotinylating erythropoietin, any of which may be used for the preparation of the erythropoietins for the uses herein. Biotin may be added to (1) the sialic acid moieties (2) carboxylate groups or (3) amino groups.

In another preferred embodiment, the lysine may be reacted with an aldehyde or reducing sugar to form an imine, which may be stabilized by reduction as with sodium cyanoborohydride to form an N-alkylated lysine such as glucitolyl lysine, or which in the case of reducing sugars may be stabilized by Amadori or Heyns rearrangement to form an alpha-deoxy alpha-amino sugar such as alpha-deoxy-alpha-fructosyllysine. As an example, preparation of a fructosyllysine-modified protein by incubation with 0.5 M glucose in sodium phosphate buffer at pH 7.4 for 60 days is described by Makita et al., 1992, J. Biol. Chem. 267:5133-5138. In another example, the lysine group may be carbamylated, such as by virtue of reaction with cyanate ion, or alkyl- or aryl-carbamylated or -thiocarbamylated with an alkyl- or aryl-isocyanate or -isothiocyanate, or it may be acylated by a reactive alkyl- or arylcarboxylic acid derivative, such as by reaction with acetic anhydride or succinic anhydride or phthalic anhydride. Exemplary are the modification of lysine groups with 4-sulfophenylisothiocyanate or with acetic anhydride, both as described in Gao et al., 1994, Proc Natl Acad Sci USA 91(25):12027-30. Lysine groups may also be trinitrophenyl modified by reaction with trinitrobenzenesulfonic acid or preferably its salts. Such methods are described below in Example 5.

At least one tyrosine residue of an erythropoietin may be modified in an aromatic ring position by an electrophilic reagent, such as by nitration or iodination. By way of non-limiting example, erythropoietin may be reacted with tetranitromethane (Nestler et al., 1985, J. Biol. Chem. 260(12):7316-21; or iodinated as described in Example 5.

At least an aspartic acid or a glutamic acid residue of an erythropoietin may be modified, such as by reaction with a carbodiimide followed by reaction with an amine such as but not limited to glycinamide. Examples of such modifications may be found in Example 5.

In another example, a tryptophan residue of an erythropoietin may be modified, such as by reaction with n-bromosuccinimide or n-chlorosuccinimide, following methods such as described in Josse et al., Chem Biol Interact 1999 May 14;119-120.

In yet another example, an erythropoietin molecule may be prepared by removing at least one amino group, such may be achieved by reaction with ninhydrin followed by reduction of the subsequent carbonyl group by reaction with borohydride.

In still a further example, an erythropoietin is provided that has at least an opening of at least one of the cystine linkages in the erythropoietin molecule by reaction with a reducing agent such as dithiothreitol, followed by reaction of the subsequent sulfhydryls with iodoacetamide, iodoacetic acid or another electrophile to prevent reformation of the disulfide linkages.

An erythropoietin is provided having at least one substitution of any one of a number of amino acids, such as a leucine, with at least one of lysine, arginine, tryptophan, tyrosine, or cysteine residues of erythropoietin, using molecular biological techniques.

A modified erythropoietin may be prepared by subjecting an erythropoietin to a limited chemical proteolysis that targets specific residues, for example, to cleave after tryptophan residues. Such resulting erythropoietin fragments are embraced herein.

As noted above, an erythropoietin useful for the purposes herein may have at least one of the aforementioned modifications, but may have more than one of the above modifications. By way of example of a modified erythropoietin with one modification to the carbohydrate portion of the molecule and one modification to the amino acid portion, a modified erythropoietin may be asialoerythropoietin and have its lysine residues biotinylated or carbamylated.

Thus, various erythropoietin molecules and pharmaceutical compositions containing them for the uses described herein are embraced. Such erythropoietin molecules include but are not limited to asialoerythropoietin, N-deglycosylated erythropoietin, O-deglycosylated erythropoietin, erythropoietin with reduced carbohydrate content, erythropoietin with altered glycosylation patterns, erythropoietin with carbohydrates oxidized then reduced, arylglyoxal-modified erythropoietin, alkylglyoxal-modified erythropoietin, 2,3-butanedione-modified erythropoietin, cyclohexanedione-modified erythropoietin, biotinylated erythropoietin, N-alkylated-lysyl-erythropoietin, glucitolyl lysine erythropoietin, alpha-deoxy-alpha-fructosyllysine-erythropoietin, carbamylated erythropoietin, acetylated erythropoietin, succinylated erythropoietin, alpha-carboxyalkyl erythropoietin, nitrated erythropoietin, iodinated erythropoietin, to name some representative yet non-limiting examples based on the teachings herein. Preferred are the aforementioned modified forms based on human erythropoietin.

Moreover, certain of the aforementioned erythropoietins are new, and the invention is directed to such compounds as well as pharmaceutical compositions comprising them. Such new erythropoietins include carbamylated asialoerythropoietin.

A variety of host-expression vector systems may be utilized to produce the erythropoietins and erythropoietin-related molecules of the invention. Such host-expression systems represent vehicles by which the erythropoietins of interest may be produced and subsequently purified, but also represent cells that may, when transformed or transfected with the appropriate nucleotide coding sequences, exhibit the modified erythropoietin gene product *in situ.* These include but are not limited to, bacteria, insect, plant, mammallian, including human host systems, such as, but not limited to, insect cell systems infected with recombinant virus expression vectors (*e.g.,* baculovirus) containing the modified erythropoietin product coding sequences; plant cell systems infected with recombinant virus expression vectors (*e.g.,* cauliflower mosaic virus, CaMV; tobacco mosaic virus, TMV) or transformed with recombinant plasmid expression vectors (*e.g.,* Ti plasmid) containing erythropoietin-related molecule coding sequences; or mammalian cell systems, including human cell systems, (*e.g.,* HT1080, COS, CHO, BHK, 293, 3T3) harboring recombinant expression constructs containing promoters derived from the genome of mammalian cells *(e.g.,* metallothionein promoter) or from mammalian viruses *(e.g.,* the adenovirus late promoter; the vaccinia virus 7.5K promoter).

In addition, a host cell strain may be chosen that modulates the expression of the inserted sequences, or modifies and processes the gene product in the specific fashion desired. Such modifications *(e.g.,* glycosylation) and processing *(e.g.,* cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells that possess the cellular machinery for proper processing of the primary transcript, glycosylation, and phosphorylation of the gene product may be used. Such mammalian host cells, including human host cells, include but are not limited to HT1080, CHO, VERO, BHK, HeLa, COS, MDCK, 293, 3T3, and WI38.

For long-term, high-yield production of recombinant proteins, stable expression is preferred. For example, cell lines that stably express the erythropoietin-related molecule gene product may be engineered. Rather than using expression vectors that contain viral origins of replication, host cells can be transformed with DNA controlled by appropriate expression control elements (*e.g.,* promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci that in turn can be cloned and expanded into cell lines. This method may advantageously be used to engineer cell lines that express the erythropoietin-related molecule gene product. Such engineered cell lines may be particularly useful in screening and evaluation of compounds that affect the endogenous activity of the erythropoietin-related molecule gene product.

Alternatively, the expression characteristic of an endogenous erythropoietin gene within a cell line or microorganism may be modified by inserting a heterologous DNA regulatory element into the genome of a stable cell line or cloned microorganism such that the inserted regulatory element is operatively linked with the endogenous erythropoietin gene. For example, an endogenous erythropoietin gene which is normally "transcriptionally silent", *i.e.,* an erythropoietin gene which is normally not expressed, or is expressed only a very low levels in a cell line, may be activated by inserting a regulatory element which is capable of promoting the expression of a normally expressed gene product in that cell line or microorganism. Alternatively, a transcriptionally silent, endogenous erythropoietin gene may be activated by insertion of a promiscuous regulatory element that works across cell types.

A heterologous regulatory element may be inserted into a stable cell line or cloned microorganism, such it is operatively linked with an endogenous erythropoietin gene, using techniques, such as targeted homologous recombination, which are well known to those of skill in the art, and described e.g., in French Patent No. 2646438 to Institut Pasteur, U.S. Patent No. 4,215,051 to Chappel; U.S. Patent No. 5,578,461 to Sherwin et al.; International Application No. PCT/US92/09627 (WO93/09222) by Selden et al.; and International Application No. PCT/US90/06436 (WO91/06667) by Skoultchi *et al.,* each of which is incorporated by reference herein in its entirety.

In one embodiment of the invention, an erythropoietin-related molecule deficient in sialic residues, or completely lacking sialic residues, may be produced in mammalian cell, including a human cell. Such cells may be engineered to be deficient in, or lacking, the enzymes that add sialic acids, *i.e.,* the β-galactoside α 2,3 sialyltransferase ("α 2,3 sialyltransferase") and the β-galactoside α 2,6 sialyltransferase ("α 2,6 sialyltransferase") activity. In one embodiment, a mammalian cell is used in which either or both the α 2,3 sialyltransferase gene and/or the α 2,6 sialyltransferase gene, is deleted. Such deletions may be constructed using gene knock-out techniques well known in the art. In another embodiment, dihydrofolate reductase (DHFR) deficient Chinese Hamster Ovary (CHO) cells are used as the host cell for the production of recombinant erythropoietin-related molecules. CHO cells do not express the enzyme α 2,6 sialyltransferase and therefore do not add sialic acid in the 2,6 linkage to N-linked oligosaccharides of glycoproteins produced in these cells. As a result, recombinant proteins produced in CHO cells lack sialic acid in the 2,6 linkage to galactose (Sasaki et al. (1987; Takeuchi et al. supra; Mutsaers et al Eur. J. Biochem. 156, 651 (1986); Takeuchi et al. J. Chromotgr. 400, 207 (1987). In one embodiment, to produce a host cell for the production of asialo-erythropoietin, the gene encoding α 2,3 sialyltransferase in CHO cells is deleted. Such α 2,3 sialyltransferase knock-out CHO cells completely lack sialyltransferase activity, and as a result, are useful for the recombinant expression and production of asialo-erythropoietin.

In another embodiment, asialo glycoproteins can be produced by interfering with sialic acid transport into the golgi apparatus e.g., Eckhardt et al., 1998, J. Biol. Chem. 273:20189-95). Using methods well known to those skilled in the art (*e.g.*, Oelmann et al., 2001, J. Biol. Chem. 276:26291-300), mutagenesis of the nucleotide sugar CMP-sialic acid transporter can be accomplished to produce mutants of Chinese hamster ovary cells. These cells cannot add sialic acid residues to glycoproteins such as erthropoietin and produce only asialoerythropoietin. Transfected mammalian cells producing erythropoietin also produce cytosolic sialidase which if it leaks into the culture medium degrades sialoerythropoietin with high efficiency (e.g., Gramer et al, 1995 Biotechnology 13:692-698). Using methods well known to those knowledgeable in the art (*e.g.,* from information provided in Ferrari et al, 1994, Glycobiology 4:367-373), cell lines can be transfected, mutated or otherwise caused to constitutively produce sialidase. In this manner, asialoerythropoietin can be produced during the manufacture of asialoerythropoietin.

In the practice of one aspect of the present invention, a pharmaceutical composition as described above containing an erythropoietin may be administerable to a mammal by any route which provides a sufficient level of an erythropoietin in the vasculature to permit translocation across an endothelial cell barrier and beneficial effects on erythropoietin-responsive cells. When used for the purpose of perfusing a tissue or organ, similar results are desired. In the instance wherein the erythropoietin is used for *ex-vivo* perfusion, the erythropoietin may be any form of erythropoietin, such as the aforementioned erythropoietins but not limited thereto an may be inclusive of native erythropoietins including human erythropoietin. In the instance where the cells or tissue is non-vascularized and/or the administration is by bathing the cells or tissue with the composition of the invention, the pharmaceutical composition provides an effective erythropoietin-responsive-cell-beneficial amount of an erythropoietin. The endothelial cell barriers across which an erythropoietin may translocate include tight junctions, perforated junctions, fenestrated junctions, and any other types of endothelial barriers present in a mammal. A preferred barrier is an endothelial cell tight junction, but the invention is not so limiting.

The aforementioned erythropoietins are useful generally for the therapeutic or prophylactic treatment of human diseases of the central nervous system or peripheral nervous system which have primarily neurological or psychiatric symptoms, ophthalmic diseases, cardiovascular diseases, cardiopulmonary diseases, respiratory diseases, kidney, urinary and reproductive diseases, gastrointestinal diseases and endocrine and metabolic abnormalities but only as far as neuronal cells/tissue are involved. In particular, such conditions and diseases include hypoxic conditions, which adversely affect excitable tissues, such as excitable tissues in the central nervous system tissue, peripheral nervous system tissue, or cardiac tissue or retinal tissue such as, for example, brain, heart, or retina/eye. Therefore, the invention can be used to treat or prevent damage to excitable tissue resulting from hypoxic conditions in a variety of conditions and circumstances. Non-limiting examples of such conditions and circumstances are provided in the table hereinbelow.

In the example of the protection of neuronal tissue pathologies treatable in accordance with the present invention, such pathologies include those which result from reduced oxygenation of neuronal tissues. Any condition which reduces the availability of oxygen to neuronal tissue, resulting in stress, damage, and finally, neuronal cell death, can be treated by the methods of the present invention. Generally referred to as hypoxia and/or ischemia, these conditions arise from or include, but are not limited to stroke, vascular occlusion, prenatal or postnatal oxygen deprivation, suffocation, choking, near drowning, carbon monoxide poisoning, smoke inhalation, trauma, including surgery and radiotherapy, asphyxia, epilepsy, hypoglycemia, chronic obstructive pulmonary disease, emphysema, adult respiratory distress syndrome, hypotensive shock, septic shock, anaphylactic shock, insulin shock, sickle cell crisis, cardiac arrest, dysrhythmia, nitrogen narcosis, and neurological deficits caused by heart-lung bypass procedures.

In one embodiment, for example, the specific EPO compositions can be administered to prevent injury or tissue damage resulting from risk of injury or tissue damage during surgical procedures, such as, for example, tumor resection or aneurysm repair. Other pathologies caused by or resulting from hypoglycemia which are treatable by the methods described herein include insulin overdose, also referred to as iatrogenic hyperinsulinemia, insulinoma, growth hormone deficiency, hypocortisolism, drug overdose, and certain tumors.

Other pathologies resulting from excitable neuronal tissue damage include seizure disorders, such as epilepsy, convulsions, or chronic seizure disorders. Other treatable conditions and diseases include diseases such as stroke, multiple sclerosis, hypotension, cardiac arrest, Alzheimer's disease, Parkinson's disease, cerebral palsy, brain or spinal cord trauma, AIDS dementia, age-related loss of cognitive function, memory loss, amyotrophic lateral sclerosis, seizure disorders, alcoholism, retinal ischemia, optic nerve damage resulting from glaucoma, and neuronal loss.

The specific compositions and methods of the invention may be used to treat conditions of, and damage to, retinal tissue. Such disorders include, but are not limited to retinal ischemia, macular degeneration, retinal detachment, retinitis pigmentosa, arteriosclerotic retinopathy, hypertensive retinopathy, retinal artery blockage, retinal vein blockage, hypotension, and diabetic retinopathy.

In another embodiment, the methods principles of the invention may be used to protect or treat injury resulting from radiation damage to excitable tissue. A further utility of the methods of the present invention is in the treatment of neurotoxin poisoning, such as domoic acid shellfish poisoning, neurolathyrism, and Guam disease, amyotrophic lateral sclerosis, and Parkinson's disease.

As mentioned above, the present invention is also directed to a method for enhancing excitable tissue function in a mammal by peripheral administration of an erythropoietin as described above. Various diseases and conditions are amenable to treatment using this method, and further, this method is useful for enhancing cognitive function in the absence of any condition or disease. These uses of the present invention are describe in further detail below and include enhancement of learning and training in both human and non-human mammals.

Conditions and diseases treatable by the methods of this aspect of the present invention directed to the central nervous system include but are not limited to mood disorders, anxiety disorders, depression, autism, attention deficit hyperactivity disorder, and cognitive dysfunction. These conditions benefit from enhancement of neuronal function. Other disorders treatable in accordance with the teachings of the present invention include sleep disruption, for example, sleep apnea and travel-related disorders; subarachnoid and aneurismal bleeds, hypotensive shock, concussive injury, septic shock, anaphylactic shock, and sequelae of various encephalitides and meningitides, for example, connective tissue disease-related cerebritides such as lupus. Other uses include prevention of or protection from poisoning by neurotoxins, such as domoic acid shellfish poisoning, neurolathyrism, and Guam disease, amyotrophic lateral sclerosis, Parkinson's disease; postoperative treatment for embolic or ischemic injury; whole brain irradiation; sickle cell crisis; and eclampsia.

A further group of conditions treatable by the methods of the present invention include mitochondrial dysfunction, of either an hereditary or acquired nature, which are the cause of a variety of neurological diseases typified by neuronal injury and death. For example, Leigh disease (subacute necrotizing encephalopathy) is characterized by progressive visual loss and encephalopathy, due to neuronal drop out, and myopathy. In these cases, defective mitochondrial metabolism fails to supply enough high energy substrates to fuel the metabolism of excitable cells. An erythropoietin receptor activity modulator optimizes failing function in a variety of mitochondrial diseases. As mentioned above, hypoxic conditions adversely affect excitable tissues. The excitable tissues include, but are not limited to, central nervous system tissue, peripheral nervous system tissue, and heart tissue. In addition to the conditions described above, the methods of the present invention are useful in the treatment of inhalation poisoning such as carbon monoxide and smoke inhalation, severe asthma, adult respiratory distress syndrome, and choking and near drowning. Further conditions which create hypoxic conditions or by other means induce excitable tissue damage include hypoglycemia that may occur in inappropriate dosing of insulin, or with insulin-producing neoplasms (insulinoma).

Various neuropsychologic disorders which are believed to originate from excitable tissue damage are treatable by the instant methods. Chronic disorders in which neuronal damage is involved and for which treatment by the present invention is provided include disorders relating to the central nervous system and/or peripheral nervous system including age-related loss of cognitive function and senile dementia, chronic seizure disorders, Alzheimer's disease, Parkinson's disease, dementia, memory loss, amyotrophic lateral sclerosis, multiple sclerosis, tuberous sclerosis, Wilson's Disease cerebral and progressive supranuclear palsy, Guam disease, Lewy body dementia, prion diseases, such as spongiform encephalopathies, e.g., Creutzfeldt-Jakob disease, Huntington's disease, myotonic dystrophy, Freidrich's ataxia and other ataxias, as well as Gilles de la Tourette's syndrome, seizure disorders such as epilepsy and chronic seizure disorder, stroke, brain or spinal cord trauma, AIDS dementia, alcoholism, autism, retinal ischemia, glaucoma, autonomic function disorders such as hypertension and sleep disorders, and neuropsychiatric disorders that include, but are not limited to schizophrenia, schizoaffective disorder, attention deficit disorder, dysthymic disorder, major depressive disorder, mania, obsessive-compulsive disorder, psychoactive substance use disorders, anxiety, panic disorder, as well as unipolar and bipolar affective disorders. Additional neuropsychiatric and neurodegenerative disorders include, for example, those listed in the American Psychiatric Association's Diagnostic and Statistical manual of Mental Disorders (DSM), the most current version of which in incorporated herein by reference in its entirety.

In another embodiment, recombinant chimeric toxin molecules comprising erythropoietin can be used for therapeutic delivery of toxins to treat a proliferative disorder, such as cancer, or viral disorder, such as subacute sclerosing panencephalitis.

The following table lists additional exemplary, non-limiting indications as to the various conditions and diseases amenable to treatment by the aforementioned erythropoietins.

| ***Cell, tissue or organ*** | ***Dysfunction or pathology*** | ***Condition or disease*** | ***Type*** |
|---|---|---|---|
| Heart | Ischemia | Coronary artery disease | Acute, chronic Stable, unstable |
| | | Myocardial infarction | Dressler's syndrome |
| | | Angina | |
| | | Congenital heart disease | Valvular Cardiomyopathy |
| | | Prinzmetal angina | |
| | | Cardiac rupture | Aneurysmatic Septal perforation |
| | | Angiitis | |
| | Arrhythmia | Tachy-, bradyarrhythmia Supraventricular, ventricular Conduction abnormalities | Stable, unstable Hypersensitive carotid sinus node |
| | Congestive heart failure | Left, right, bi-ventricular | Cardiomyopathies, such as idiopathic familial, infective, metabolic, storage disease, deficiencies, connective tissue disorder, infiltration and granulomas, neurovascular |
| | | Myocarditis | Autoimmune, infective, idiopathic |
| | | Cor pulmonale | |
| | Blunt and penetrating trauma | | |
| | Toxins | Cocaine | |
| | | | |
| Vascular | Hypertension | Primary, secondary | |
| | Decompression sickness | | |
| | Fibromuscular hyperplasia | | |
| | Aneurysm | Dissecting, ruptured, enlarging | |
| | | | |
| Lungs | Obstructive | Asthma Chronic bronchitis, Emphysema and airway obstruction | |
| | Ischemic lung disease | Pulmonary embolism, Pulmonary thrombosis, Fat embolism | |
| | Environmental lung diseases | | |
| | Ischemic lung disease | Pulmonary embolism Pulmonary thrombosis | |
| | Interstitial lung disease | Idiopathic pulmonary fibrosis | |
| | Congenital | Cystic fibrosis | |
| | Cor pulmonale | | |
| | Trauma | | |
| | Pneumonia and pneumonitides | Infectious, parasitic, toxic, traumatic, burn, aspiration | |
| | Sarcoidosis | | |
| | | | |
| Pancreas | Endocrine | Diabetes mellitus, type I and II | Beta cell failure, dysfunction Diabetic neuropathy |
| | | Other endocrine cell failure of the pancreas | |
| | Exocrine | Exocrine pancreas failure | pancreatitis |
| | | | |
| Bone | Osteopenia | Primary secondary | Hypogonadism immobilisation |
| | | | Postmenopausal Age-related Hyperparathyroidism Hyperthyroidism Calcium, magnesium, phosphorus and/or vitamin D deficiency |
| | Osteomyelitis | | |
| | Avascular necrosis | | |
| | Trauma | | |
| | Paget's disease | | |
| | | | |
| Skin | Alopecia | Areata Totalis | Primary Secondary Male pattern baldness |
| | Vitiligo | Localized generalized | Primary secondary |
| | Diabetic ulceration | | |
| | Peripheral vascular disease | | |
| | Burn injuries | | |
| | | | |
| Autoimmune disorders | Lupus erythematodes, Sjiogren, Rheumatoid arthritis, Glomerulonephritis, Angiitis | | |
| | Langerhan's histiocytosis | | |
| | | | |
| Eye | Optic neuritis | | |
| | Blunt and penetrating injuries, Infections, Sarcoid, Sickle C disease, Retinal detachment, Temporal arteritis | | |
| | | | |
| Embryonic and fetal disorders | Asphyxia | | |
| | Ischemia | | |
| | | | |
| CNS | Chronic fatigue syndrome, acute and chronic hypoosmolar and hyperosmolar syndromes, AIDS Dementia, Electrocution | | |
| | Encephalitis | Rabies, Herpes | |
| | Meningitis | | |
| | Subdural hematoma | | |
| | Nicotine addiction | | |
| | Drug abuse and withdrawal | Cocaine, heroin, crack, marijuana, LSD, PCP, poly-drug abuse, ecstasy, opioids, sedative hypnotics, amphetamines, caffeine | |
| | Obsessive-compulsive disorders | | |
| | Spinal stenosis, Transverse myelitis, Guillian Barre, Trauma, Nerve root compression, Tumoral compression, Heat stroke | | |
| ENT | Tinnitus Meuniere's syndrome Hearing loss | | |
| | Traumatic injury, barotrauma | | |
| Kidney | Renal failure | Acute, chronic | Vascular/ischemic, interstitial disease, diabetic kidney disease, nephrotic syndromes, infections |
| | Henoch S. Purpura | | |
| | | | |
| Striated muscle | Autoimmune disorders | Myasthenia gravis Dermatomyositis Polymyositis | |
| | | | |
| | Myopathies | Inherited metabolic, endocrine and toxic | |
| | Heat stroke | | |
| | Crush injury | | |
| | Rhabdomylosis | | |
| | Mitochondrial disease | | |
| | Infection | Necrotizing fasciitis | |
| | | | |
| Sexual dysfunction | Central and peripheral | Impotence secondary to medication | |
| | | | |
| Liver | hepatitis | Viral, bacterial, parasitic | |
| | Ischemic disease | | |
| | Cirrhosis, fatty liver | | |
| | Infiltrative/metabolic diseases | | |
| | | | |
| Gastrointestinal | Ischemic bowel disease | | |
| | Inflammatory bowel disease | | |
| | Necrotizing enterocolitis | | |
| | | | |
| Organ transplantation | Treatment of donor and recipient | | |
| | | | |
| Reproductive tract | infertility | Vascular Autoimmune Uterine abnormalities Implantation disorders | |
| | | | |
| Endocrine | Glandular hyper- and hypofunction | | |

Accordingly, this invention generally provides therapeutic or prophylactic treatment of the consequences of mechanical trauma or of human diseases. Therapeutic or prophylactic treatment for diseases, disorders or conditions of the CNS and/or peripheral nervous system are preferred. Therapeutic or prophylactic treatment for diseases, disorders or conditions which have a psychiatric component is provided. Therapeutic or prophylactic treatment for diseases, disorders or conditions including but not limited to those having an ophthalmic, cardiovascular, cardiopulmonary, respiratory, kidney, urinary, reproductive, gastrointestinal, endocrine, or metabolic component is provided.

In one embodiment, such a pharmaceutical composition of an erythropoietin may be administered systemically to protect or enhance the target cells, tissue or organ. Such administration may be parenterally, via inhalation, or transmucosally, *e.g.,* orally, nasally, rectally, intravaginally, sublingually, submucosally or transdermally. Preferably, administration is parenteral, *e.g.,* via intravenous or intraperitoneal injection, and also including, but is not limited to, intra-arterial, intramuscular, intradermal and subcutaneous administration.

For other routes of administration, such as by use of a perfusate, injection into an organ, or other local administration, a pharmaceutical composition will be provided which results in similar levels of an erythropoietin as described above. A level of about 15pM -30 nM is preferred.

The pharmaceutical compositions of the invention may comprise a therapeutically effective amount of a compound, and a pharmaceutically acceptable carrier. In a specific embodiment, the term "pharmaceutically acceptable" means approved by a regulatory agency of the Federal or a state government or listed in the U.S. Pharmacopeia or other generally recognized foreign pharmacopeia for use in animals, and more particularly in humans. The term "carrier" refers to a diluent, adjuvant, excipient, or vehicle with which the therapeutic is administered. Such pharmaceutical carriers can be sterile liquids, such as saline solutions in water and oils, including those of petroleum, animal, vegetable or synthetic origin, such as peanut oil, soybean oil, mineral oil, sesame oil and the like. A saline solution is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. The compounds of the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc. Examples of suitable pharmaceutical carriers are described in "Remington's Pharmaceutical Sciences" by E.W. Martin. Such compositions will contain a therapeutically effective amount of the compound, preferably in purified form, together with a suitable amount of carrier so as to provide the form for proper administration to the patient. The formulation should suit the mode of administration.

Pharmaceutical compositions adapted for oral administration may be provided as capsules or tablets; as powders or granules; as solutions, syrups or suspensions (in aqueous or non-aqueous liquids); as edible foams or whips; or as emulsions. Tablets or hard gelatine capsules may comprise lactose, starch or derivatives thereof, magnesium stearate, sodium saccharine, cellulose, magnesium carbonate, stearic acid or salts thereof. Soft gelatine capsules may comprise vegetable oils, waxes, fats, semi-solid, or liquid polyols etc. Solutions and syrups may comprise water, polyols and sugars.

An active agent intended for oral administration may be coated with or admixed with a material that delays disintegration and/or absorption of the active agent in the gastrointestinal tract (*e.g.,* glyceryl monostearate or glyceryl distearate may be used). Thus, the sustained release of an active agent may be achieved over many hours and, if necessary, the active agent can be protected from being degraded within the stomach. Pharmaceutical compositions for oral administration may be formulated to facilitate release of an active agent at a particular gastrointestinal location due to specific pH or enzymatic conditions.

Pharmaceutical compositions adapted for transdermal administration may be provided as discrete patches intended to remain in intimate contact with the epidermis of the recipient for a prolonged period of time. Pharmaceutical compositions adapted for topical administration may be provided as ointments, creams, suspensions, lotions, powders, solutions, pastes, gels, sprays, aerosols or oils. For topical administration to the skin, mouth, eye or other external tissues a topical ointment or cream is preferably used. When formulated in an ointment, the active ingredient may be employed with either a paraffinic or a water-miscible ointment base. Alternatively, the active ingredient may be formulated in a cream with an oil-in-water base or a water-in-oil base. Pharmaceutical compositions adapted for topical administration to the eye include eye drops. In these compositions, the active ingredient can be dissolved or suspended in a suitable carrier, *e.g.,* in an aqueous solvent. Pharmaceutical compositions adapted for topical administration in the mouth include lozenges, pastilles and mouthwashes.

Pharmaceutical compositions adapted for nasal and pulmonary administration may comprise solid carriers such as powders (preferably having a particle size in the range of 20 to 500 microns). Powders can be administered in the manner in which snuff is taken, *i.e.,* by rapid inhalation through the nose from a container of powder held close to the nose. Alternatively, compositions adopted for nasal administration may comprise liquid carriers, *e.g.,* nasal sprays or nasal drops. Alternatively, inhalation directly into the lungs may be accomplished by inhalation deeply or installation through a mouthpiece into the oropharynx. These compositions may comprise aqueous or oil solutions of the active ingredient. Compositions for administration by inhalation may be supplied in specially adapted devices including, but not limited to, pressurized aerosols, nebulizers or insufflators, which can be constructed so as to provide predetermined dosages of the active ingredient. In a preferred embodiment, pharmaceutical compositions of the invention are administered into the nasal cavity directly or into the lungs via the nasal cavity or oropharynx.

Pharmaceutical compositions adapted for rectal administration may be provided as suppositories or enemas. Pharmaceutical compositions adapted for vaginal administration may be provided as pessaries, tampons, creams, gels, pastes, foams or spray formulations.

Pharmaceutical compositions adapted for parenteral administration include aqueous and non-aqueous sterile injectable solutions or suspensions, which may contain antioxidants, buffers, bacteriostats and solutes that render the compositions substantially isotonic with the blood of an intended recipient. Other components that may be present in such compositions include water, alcohols, polyols, glycerine and vegetable oils, for example. Compositions adapted for parenteral administration may be presented in unit-dose or multi-dose containers, for example sealed ampules and vials, and may be stored in a freeze-dried (lyophilized) condition requiring only the addition of a sterile liquid carrier, *e.g.,* sterile saline solution for injections, immediately prior to use. Extemporaneous injection solutions and suspensions may be prepared from sterile powders, granules and tablets. In one embodiment, an autoinjector comprising an injectable solution of an erythropoietin may be provided for emergency use by ambulances, emergency rooms, and battlefield situations, and even for self-administration in a domestic setting, particularly where the possibility of traumatic amputation may occur, such as by imprudent use of a lawn mower. The likelihood that cells and tissues in a severed foot or toe will survive after reattachment may be increased by administering an erythropoietin to multiple sites in the severed part as soon as practicable, even before the arrival of medical personnel on site, or arrival of the afflicted individual with severed toe in tow at the emergency room.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically-sealed container such as an ampule or sachette indicating the quantity of active agent. Where the composition is to be administered by infusion, it can be dispensed with an infusion bottle containing sterile pharmaceutical grade water or saline. Where the composition is administered by injection, an ampule of sterile saline can be provided so that the ingredients may be mixed prior to administration.

Suppositories generally contain active ingredient in the range of 0.5% to 10% by weight; oral formulations preferably contain 10% to 95% active ingredient.

A perfusate composition may be provided for use in transplanted organ baths, for in situ perfusion, or for administration to the vasculature of an organ donor prior to organ harvesting. Such pharmaceutical compositions may comprise levels of an erythropoietin or a form of an erythropoietin not suitable for acute or chronic, local or systemic administration to an individual, but will serve the functions intended herein in a cadaver, organ bath, organ perfusate, or in situ perfusate prior to removing or reducing the levels of the erythropoietin contained therein before exposing or returning the treated organ or tissue to regular circulation. The erythropoietin for this aspect of the invention may be any erythropoietin, such as naturally-occurring forms such as human erythropoietin, or any of the erythropoietins hereinabove described, such as asialoerythropoietin and phenylglyoxal-erythropoietins, as non-limiting examples.

The specification also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

In another embodiment, for example, erythropoietin can be delivered in a controlled-release system. For example, the polypeptide may be administered using intravenous infusion, an implantable osmotic pump, a transdermal patch, liposomes, or other modes of administration. In one embodiment, a pump may be used (see Langer, *supra;* Sefton, 1987, CRC Crit. Ref. Biomed. Eng. 14:201; Buchwald et al., 1980, Surgery 88:507; Saudek et al., 1989, N. Engl. J. Med. 321:574). In another embodiment, the compound can be delivered in a vesicle, in particular a liposome (*see* Langer, Science 249:1527-1533 (1990); Treat et al., in Liposomes in the Therapy of Infectious Disease and Cancer, Lopez-Berestein and Fidler (eds.), Liss, New York, pp. 353-365 (1989); WO 91/04014; U.S. Patent No. 4,704,355; Lopez-Berestein, *ibid.,* pp. 317-327; *see* generally *ibid*.). In another embodiment, polymeric materials can be used [see Medical Applications of Controlled Release, Langer and Wise (eds.), CRC Press: Boca Raton, Florida, 1974; Controlled Drug Bioavailability, Drug Product Design and Performance, Smolen and Ball (eds.), Wiley: New York (1984); Ranger and Peppas, J. Macromol. Sci. Rev. Macromol. Chem. 23:61, 1953; see also Levy et al., 1985, Science 228:190; During et al., 1989, Ann. Neurol. 25:351; Howard et al., 1989, J. Neurosurg. 71:105).

In yet another embodiment, a controlled release system can be placed in proximity of the therapeutic target, *i.e.,* the target cells, tissue or organ, thus requiring only a fraction of the systemic dose (see, e.g., Goodson, pp. 115-138 in Medical Applications of Controlled Release, vol. 2, supra, 1984). Other controlled release systems are discussed in the review by Langer (1990, Science 249:1527-1533).

In another embodiment, erythropoietin, as properly formulated, can be administered by nasal, oral, rectal, vaginal, or sublingual administration.

In a specific embodiment, it may be desirable to administer the erythropoietin compositions of the invention locally to the area in need of treatment; this may be achieved by, for example, and not by way of limitation, local infusion during surgery, topical application, *e.g.,* in conjunction with a wound dressing after surgery, by injection, by means of a catheter, by means of a suppository, or by means of an implant, said implant being of a porous, non-porous, or gelatinous material, including membranes, such as silastic membranes, or fibers.

Selection of the preferred effective dose will be determined by a skilled artisan based upon considering several factors which will be known to one of ordinary skill in the art. Such factors include the particular form of erythropoietin, and its pharmacokinetic parameters such as bioavailability, metabolism, half-life, etc., which will have been established during the usual development procedures typically employed in obtaining regulatory approval for a pharmaceutical compound. Further factors in considering the dose include the condition or disease to be treated or the benefit to be achieved in a normal individual, the body mass of the patient, the route of administration, whether administration is acute or chronic, concomitant medications, and other factors well known to affect the efficacy of administered pharmaceutical agents. Thus the precise dosage should be decided according to the judgment of the practitioner and each patient's circumstances, *e.g.,* depending upon the condition and the immune status of the individual patient, according to standard clinical techniques.

In another aspect of the invention, a perfusate or perfusion solution is provided for perfusion and storage of organs for transplant, the perfusion solution including an amount of an erythropoietin effective to protect erythropoietin-responsive cells and associated cells, tissues or organs. Transplant includes but is not limited to xenotransplantation, where a organ (including cells, tissue or other bodily part) is harvested from one donor and transplanted into a different recipient; and autotransplant, where the organ is taken from one part of a body and replaced at another, including bench surgical procedures, in which an organ may be removed, and while *ex vivo,* resected, repaired, or otherwise manipulated, such as for tumor removal, and then returned to the original location. In one embodiment, the perfusion solution is the University of Wisconsin (UW) solution (U.S. Patent No. 4,798,824) which contains from about 1 to about 25 U/ml erythropoietin, 5% hydroxyethyl starch (having a molecular weight of from about 200,000 to about 300,000 and substantially free of ethylene glycol, ethylene chlorohydrin, sodium chloride and acetone); 25mM KH₂PO_{4;} 3mM glutathione; 5mM adenosine; 10mM glucose; 10mM HEPES buffer; 5mM magnesium gluconate; 1.5mM CaCl_{2;} 105mM sodium gluconate; 200,000 units penicillin; 40 units insulin; 16mg Dexamethasone; 12mg Phenol Red; and has a pH of 7.4-7.5 and an osmolality of about 320 mOSm/l. The solution is used to maintain cadaveric kidneys and pancreases prior to transplant. Using the solution, preservation can be extended beyond the 30-hour limit recommended for cadaveric kidney preservation. This particular perfusate is merely illustrative of a number of such solutions that can be adapted for the present use by inclusion of an effective amount of an erythropoietin. In a further embodiment, the perfusate solution contains from about 5 to about 35 U/ml erythropoietin, or from about 10 to about 30 U/ml erythropoietin.

While the preferred recipient of an erythropoietin for the purposes herein throughout is a human, the methods herein apply equally to other mammals, particularly domesticated animals, livestock, companion and zoo animals. However, the invention is not so limiting and the benefits can be applied to any mammal.

In further aspects of the *ex-vivo* invention, any erythropoietin described above, or combinations thereof can be used.

In another aspect of the invention, methods and compositions for enhancing the viability of cells, tissues or organs which are not isolated from the vasculature by an endothelial cell barrier are provided by exposing the cells, tissue or organs directly to a pharmaceutical composition comprising an erythropoietin, or administering or contacting an erythropoietin-containing pharmaceutical composition to the vasculature of the tissue or organ. Enhanced activity of erythropoietin-responsive cells in the treated tissue or organ are responsible for the positive effects exerted.

As described above, the invention is based, in part, on the discovery that erythropoietin molecules can be transported from the luminal surface to the basement membrane surface of endothelial cells of the capillaries of organs with endothelial cell tight junctions, including, for example, the brain, retina, and testis. Thus, erythropoietin-responsive cells across the barrier are susceptible targets for the beneficial effects of erythropoietin, and others cell types or tissues or organs that contain and depend in whole or in part on erythropoietin-responsive cells therein are targets for the methods of the invention. While not wishing to be bound by any particular theory, after transcytosis of erythropoietin, erythropoietin can interact with an erythropoietin receptor on an erythropoietin-responsive cell, for example, neuronal, retinal, muscle, heart, lung, liver, kidney, small intestine, adrenal cortex, adrenal medulla, capillary endothelial, testes, ovary, or endometrial cell, and receptor binding can initiate a signal transduction cascade resulting in the activation of a gene expression program within the erythropoietin-responsive cell or tissue, resulting in the protection of the cell or tissue, or organ, from damage, such as by toxins, chemotherapeutic agents, radiation therapy, hypoxia, *etc.* Thus, methods for protecting erythropoietin-responsive cell-containing tissue from injury or hypoxic stress, and enhancing the function of such tissue are described in detail hereinbelow.

In the practice of one embodiment of the invention, a mammalian patient is undergoing systemic chemotherapy for cancer treatment, including radiation therapy, which commonly has adverse effects such as nerve, lung, heart, ovarian or testicular damage. Administration of a pharmaceutical composition comprising an erythropoietin as described above is performed prior to and during chemotherapy and/or radiation therapy, to protect neuronal tissues and organs from damage by the chemotherapeutic agent, such as to protect the testes. Treatment may be continued until circulating levels of the chemotherapeutic agent have fallen below a level of potential danger to the mammalian body.

In the practice of another embodiment of the invention, various organs were planned to be harvested from a victim of an automobile accident for transplant into a number of recipients, some of which required transport for an extended distance and period of time. Prior to organ harvesting, the victim was infused with a pharmaceutical composition comprising an erythropoietin as described herein. Harvested organs for shipment were perfused with a perfusate containing erythropoietin as described herein, and stored in a bath comprising erythropoietin. Certain organs were continuously perfused with a pulsatile perfusion device, utilizing a perfusate containing an erythropoietin in accordance with the present invention. Minimal deterioration of organ function occurred during the transport and upon implant and reperfusion of the organs *in situ.*

In another embodiment of the invention, a surgical procedure to repair a heart valve required temporary cardioplegia and arterial occlusion. Prior to surgery, the patient was infused with 500 U erythropoietin per kg body weight. Such treatment prevented hypoxic ischemic cellular damage, particularly after reperfusion.

In another embodiment of the invention, in any surgical procedure, such as in cardiopulmonary bypass surgery, a naturally-occurring erythropoietin or any erythropoietin of the invention can be used. In one embodiment, administration of a pharmaceutical composition comprising an erythropoietin as described above is performed prior to, during, and/or following the bypass procedure, to protect the function of neuronal tissue brain, heart, and other other organs.

In the foregoing examples in which an erythropoietin of the invention, namely said modified erythropoietin, is used for *ex-vivo* applications, or to treat erythropoietin-responsive cells such as neuronal tissue, the invention provides a pharmaceutical composition in dosage unit form adapted for protection or enhancement of erythropoietin-responsive cells, tissues or organs distal to the vasculature which comprises, per dosage unit, an effective non-toxic amount within the range from about 50,000 to 500,000 Units, 60,000 to 500,000 Units, 70,000 to 500,000 Units, 80,000 to 500,000 Units, 90,000 to 500,000 Units, 100,000 to 500,000 Units, 150,000 to 500,000 Units, 200,000 to 500,000 Units, 250,000 to 500,000 Units, 300,000 to 500,000 Units, 350,000 to 500,000 Units, 400,000 to 500,000 Units, or 450,000 to 500,000 Units of said erythropoietin, pharmaceutically acceptable carrier. In a preferred embodiment, the effective non-toxic amount of erythropoietin is within the range from about 50,000 to 500,000 Units. The erythropoietin in the aforementioned composition is non-erythropoietic.

In a further aspect of the invention, erythropoietin administration was found to restore cognitive function in animals having undergone brain trauma. After a delay of either 5 days or 30 days, administration of erythropoietin was still able to restore function as compared to sham-treated animals, indicating the ability of an erythropoietin to regenerate or restore brain activity. Thus, the invention is also directed to the use of an erythropoietin for the preparation of a pharmaceutical composition for the treatment of brain trauma and other cognitive dysfunctions, including treatment well after the injury (e.g. three days, five days, a week, a month, or longer). The invention is also directed to a method for the treatment of cognitive dysfunction following injury by administering an effective amount of an erythropoietin. Any erythropoietin as described herein may be used for this aspect of the invention.

Furthermore, this restorative aspect of the invention is directed to the use of any of the erythropoietins herein for the preparation of a pharmaceutical composition for the restoration of neuronal cellular, tissue or organ dysfunction, wherein treatment is initiated after, and well after, the initial insult responsible for the dysfunction. Moreover, treatment using erythropoietins of the invention can span the course of the disease or condition during the acute phase as well as a chronic phase.

The erythropoietin useful for the purposes herein is nonerythropoietic, *i.e.,* it is capable of exerting the activities described herein but not causing an increase in hemoglobin concentration or hematocrit. Such a non-erythropoietic form of erythropoietin is preferred in instances wherein the methods of the present invention are intended to be provided chronically. In another embodiment, an erythropoietin is given at a dose greater than that necessary to maximally stimulate erythropoiesis. As noted above, an erythropoietin of the invention does not necessarily have erythropoietic activity, and therefore the above dosages expressed in hematopoietic units is merely exemplary for erythropoietins that are erythropoietic; hereinabove molar equivalents for dosages are provided which are applicable to any erythropoietin.

A reference aspect is further directed to a method for facilitating the transport of a molecule across an endothelial cell barrier in a mammal by administering a composition which comprises the particular molecule in association with an erythropoietin as described hereinabove. As described above, tight junctions between endothelial cells in certain organs in the body create a barrier to the entry of certain molecules. For treatment of various conditions within the barriered organ, means for facilitating passage of pharmaceutical agents is desired. An erythropoietin of the specification is useful as a carrier for delivering other molecules across the blood-brain and other similar barriers. A composition comprising a molecule desirous of crossing the barrier with erythropoietin is prepared, and peripheral administration of the composition results in the transcytosis of the composition across the barrier. The association between the molecule to be transported across the barrier and the erythropoietin may be a labile covalent bond, in which case the molecule is released from association with the erythropoietin after crossing the barrier. If the desired pharmacological activity of the molecule is maintained or unaffected by association with erythropoietin, such a complex can be administered.

The skilled artisan will be aware of various means for associating molecules with an erythropoietin of the invention and the other agents described above, by covalent, non-covalent, and other means; furthermore, evaluation of the efficacy of the composition can be readily determined in an experimental system. Association of molecules with an erythropoietin may be achieved by any number of means, including labile, covalent binding, cross-linking, etc. Biotin/avidin interactions may be employed. As mentioned above, a hybrid molecule may be prepared by recombinant or synthetic means, for example, which includes both the domain of the molecule with desired pharmacological activity and the domain responsible for erythropoietin receptor activity modulation.

A molecule may be conjugated to an erythropoietin through a polyfunctional molecule, *i.e.,* a polyfunctional crosslinker. As used herein, the term "polyfunctional molecule" encompasses molecules having one functional group that can react more than one time in succession, such as formaldehyde, as well as molecules with more than one reactive group. As used herein, the term "reactive group" refers to a functional group on the crosslinker that reacts with a functional group on a molecule (*e.g.*, peptide, protein, carbohydrate, nucleic acid, particularly a hormone, antibiotic, or anti-cancer agent to be delivered across an endothelial cell barrier) so as to form a covalent bond between the cross-linker and that molecule. The term "functional group" retains its standard meaning in organic chemistry. The polyfunctional molecules which can be used are preferably biocompatible linkers, *i.e.,* they are noncarcinogenic, nontoxic, and substantially non-immunogenic *in vivo.* Polyfunctional cross-linkers such as those known in the art and described herein can be readily tested in animal models to determine their biocompatibility. The polyfunctional molecule is preferably bifunctional. As used herein, the term "bifunctional molecule" refers to a molecule with two reactive groups. The bifunctional molecule may be heterobifunctional or homobifunctional. A heterobifunctional cross-linker allows for vectorial conjugation. It is particularly preferred for the polyfunctional molecule to be sufficiently soluble in water for the cross-linking reactions to occur in aqueous solutions such as in aqueous solutions buffered at pH 6 to 8, and for the resulting conjugate to remain water soluble for more effective bio-distribution. Typically, the polyfunctional molecule covalently bonds with an amino or a sulfhydryl functional group. However, polyfunctional molecules reactive with other functional groups, such as carboxylic acids or hydroxyl groups, are contemplated in the present invention.

The homobifunctional molecules have at least two reactive functional groups, which are the same. The reactive functional groups on a homobifunctional molecule include, for example, aldehyde groups and active ester groups. Homobifunctional molecules having aldehyde groups include, for example, glutaraldehyde and subaraldehyde. The use of glutaraldehyde as a cross-linking agent was disclosed by Poznansky et al., Science 223, 1304-1306 (1984). Homobifunctional molecules having at least two active ester units include esters of dicarboxylic acids and N-hydroxysuccinimide. Some examples of such N-succinimidyl esters include disuccinimidyl suberate and dithio-bis-(succinimidyl propionate), and their soluble bis-sulfonic acid and bis-sulfonate salts such as their sodium and potassium salts. These homobifunctional reagents are available from Pierce, Rockford, Illinois.

The heterobifunctional molecules have at least two different reactive groups. The reactive groups react with different functional groups, *e.g.,* present on the erythropoietin and the molecule. These two different functional groups that react with the reactive group on the heterobifunctional cross-linker are usually an amino group, *e.g.,* the epsilon amino group of lysine; a sulfhydryl group, *e.g.,* the thiol group of cysteine; a carboxylic acid, *e.g.,* the carboxylate on aspartic acid; or a hydroxyl group, *e.g.,* the hydroxyl group on serine.

Of course, the various erythropoietin molecules of the invention may not have suitable reactive groups available for use with certain cross-linking agent; however, one of skill in the art will be amply aware of the choice of cross-linking agents based on the available groups for cross-linking in an erythropoietin of the invention.

When a reactive group of a heterobifunctional molecule forms a covalent bond with an amino group, the covalent bond will usually be an amido or imido bond. The reactive group that forms a covalent bond with an amino group may, for example, be an activated carboxylate group, a halocarbonyl group, or an ester group. The preferred halocarbonyl group is a chlorocarbonyl group. The ester groups are preferably reactive ester groups such as, for example, an N-hydroxysuccinimide ester group.

The other functional group typically is either a thiol group, a group capable of being converted into a thiol group, or a group that forms a covalent bond with a thiol group. The covalent bond will usually be a thioether bond or a disulfide. The reactive group that forms a covalent bond with a thiol group may, for example, be a double bond that reacts with thiol groups or an activated disulfide. A reactive group containing a double bond capable of reacting with a thiol group is the maleimido group, although others, such as acrylonitrile, are also possible. A reactive disulfide group may, for example, be a 2-pyridyldithio group or a 5,5'-dithio-bis-(2-nitrobenzoic acid) group. Some examples of heterobifunctional reagents containing reactive disulfide bonds include N-succinimidyl 3-(2-pyridyl-dithio)propionate (Carlsson, et al., 1978, Biochem J., 173:723-737), sodium S-4-succinimidyloxycarbonyl-alpha-methylbenzylthiosulfate, and 4-succinimidyloxycarbonyl-alpha-methyl-(2-pyridyldithio)toluene. N-succinimidyl 3-(2-pyridyldithio)propionate is preferred. Some examples of heterobifunctional reagents comprising reactive groups having a double bond that reacts with a thiol group include succinimidyl 4-(N-maleimidomethyl)cyclohexane-1-carboxylate and succinimidyl m-maleimidobenzoate.

Other heterobifunctional molecules include succinimidyl 3-(maleimido)propionate, sulfosuccinimidyl 4-(p-maleimido-phenyl)butyrate, sulfosuccinimidyl 4-(N-maleimidomethyl-cyclohexane)-1-carboxylate; maleimidobenzoyl-N-hydroxy-succinimide ester. The sodium sulfonate salt of succinimidyl m-maleimidobenzoate is preferred. Many of the above-mentioned heterobifunctional reagents and their sulfonate salts are available from Pierce Chemical Co., Rockford, Illinois USA.

The need for the above-described conjugated to be reversible or labile may be readily determined by the skilled artisan. A conjugate may be tested in vitro for both the erythropoietin, and for the desirable pharmacological activity. If the conjugate retains both properties, its suitability may then be tested *in vivo.* If the conjugated molecule requires separation from the erythropoietin for activity, a labile bond or reversible association with erythropoietin will be preferable. The lability characteristics may also be tested using standard in vitro procedures before *in vivo* testing.

Additional information regarding how to make and use these as well as other polyfunctional reagents may be obtained from the following publications or others available in the art:
Carlsson, J. et al., 1978, Biochem. J. 173:723-737.
Cumber, J.A. et al., 1985, Methods in Enzymology 112:207-224.
Jue, R. et al., 1978, Biochem 17:5399-5405.
Sun, T.T. et al., 1974, Biochem. 13:2334-2340.
Blattler, W.A. et al., 1985, Biochem. 24:1517-152.
Liu, F.T. et al., 1979, Biochem. 18:690-697.
Youle, R.J. and Neville, D.M. Jr., 1980, Proc. Natl. Acad. Sci. U.S.A. 77:5483-5486.
Lerner, R.A. et al., 1981, Proc. Natl. Acad. Sci. U.S.A. 78:3403-3407.
Jung, S.M. and Moroi, M., 1983, Biochem. Biophys. Acta 761:162.
Caulfield, M.P. et al., 1984, Biochem. 81:7772-7776.
Staros, J.V., 1982, Biochem. 21:3950-3955.
Yoshitake, S. et al., 1979, Eur. J. Biochem. 101:395-399.
Yoshitake, S. et al., 1982, J. Biochem. 92:1413-1424.
Pilch, P.F. and Czech, M.P., 1979, J. Biol. Chem. 254:3375-3381.
Novick, D. et al., 1987, J. Biol. Chem. 262:8483-8487.
Lomant, A.J. and Fairbanks, G., 1976, J. Mol. Biol. 104:243-261.
Hamada, H. and Tsuruo, T., 1987, Anal. Biochem. 160:483-488.
Hashida, S. et al., 1984, J. Applied Biochem. 6:56-63.

Additionally, methods of cross-linking are reviewed by Means and Feeney, 1990, Bioconjugate Chem. 1:2-12.

Barriers which are crossed by the above-described methods and compositions of the present invention include but are not limited to the blood-brain barrier, the blood-eye barrier, the blood-testes barrier, the blood-ovary barrier, and the blood-uterus barrier.

Candidate molecules for transport across an endothelial cell barrier include, for example, hormones such as growth hormone, neurotrophic factors, antibiotics or antifungals such as those normally excluded from the brain and other barriered organs, peptide radiopharmaceuticals, antisense drugs, antibodies against biologically-active agents, pharmaceuticals, and anti-cancer agents. Non-limiting examples of such molecules include growth hormone, nerve growth factor (NGF), brain-derived neurotrophic factor (BDNF), ciliary neurotrophic factor (CNTF), basic fibroblast growth factor (bFGF), transforming growth factor β1 (TGFβ1), transforming growth factor β2 (TGFβ2), transforming growth factor β3 (TGFβ3), interleukin 1, interleukin 2, interleukin 3, and interleukin 6, AZT, antibodies against tumor necrosis factor, and immunosuppressive agents such as cyclosporin.

The present specification is also directed to a composition comprising a molecule to be transported via transcytosis across a endothelial cell tight junction barrier and an erythropoietin as described above. The specification is further directed to the use of a conjugate between a molecule and an erythropoietin as described above for the preparation of a pharmaceutical composition for the delivery of the molecule across a barrier as described above.

The present invention may be better understood by reference to the following non-limiting Examples, which are provided as exemplary of the invention. The following examples are presented in order to more fully illustrate the preferred embodiments of the invention. They should in no way be construed, however, as limiting the broad scope of the invention.

### Example 1

### ERYTHROPOIETIN CROSSES THE BLOOD-CEREBROSPINAL FLUID TIGHT BARRIER

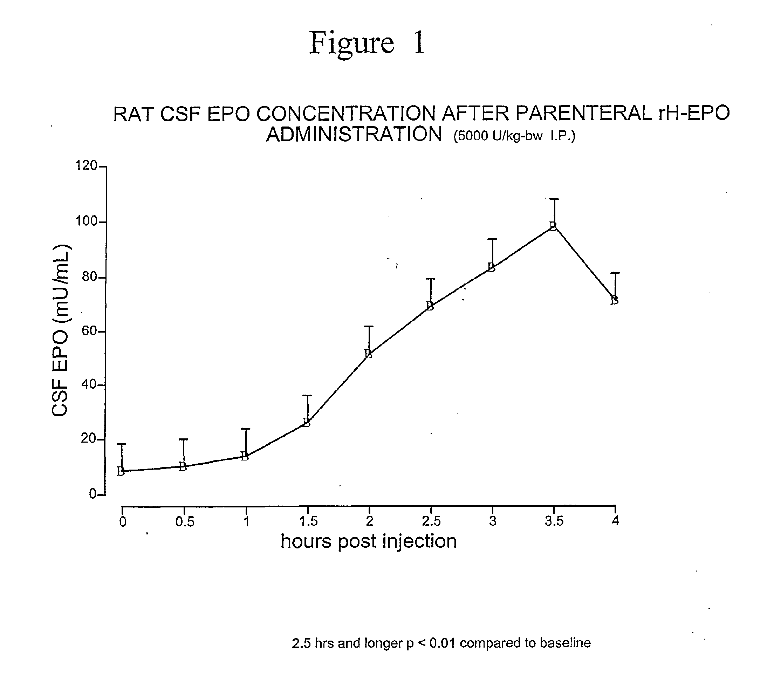

Adult male Sprague-Dawley rats were anesthetized and administered recombinant human erythropoietin intraperitoneally. Cerebrospinal fluid was sampled from the cisterna magna at 30 minute intervals up to 4 hrs and the erythropoietin concentration determined using a sensitive and specific enzyme-linked immunoassay. As illustrated in **Figure 1****,** the baseline erythropoietin concentration in CSF is 8 mU/ml. After a delay of several hours, the levels of erythropoietin measured in the CSF begin to rise and by 2.5 hours and later are significantly different from the baseline concentration at the p < 0.01 level. The peak level of about 100 mU/ml is within the range known to exert protective effects *in vitro* (0.1 to 100 mU/ml). The time to peak occurs at about 3.5 hrs, which is delayed significantly from the peak serum levels (less than 1 hr). The results of this experiment illustrate that significant levels of erythropoietin can be accomplished across a tight cellular junction by bolus parenteral administration of erythropoietin at appropriate concentrations.

### Example 2

### MAINTENANCE OF FUNCTION IN HEART PREPARED FOR TRANSPLANTATION

Wistar male rats weighing 300 to 330g are given erythropoietin (5000 U/kg body weight) or vehicle 24h prior to removal of the heart for *ex vivo* studies, done in accordance with the protocol ofDelcayre et al., 1992, Amer. J. Physiol. 263:H1537-45. Animals are sacrificed with pentobarbital (0.3mL), and intravenously heparinized (0.2mL). The hearts are initially allowed to equilibrate for 15 min The left ventricular balloon is then inflated to a volume that gives an end-diastolic pressure of 8 mm Hg. A left ventricular pressure-volume curve is constructed by incremental inflation of the balloon volume by 0.02 ml aliquots. Zero volume is defined as the point at which the left ventricular end-diastolic pressure is zero. On completion of the pressure-volume curve, the left ventricular balloon is deflated to set end-diastolic pressure back to 8mmHg and the control period is pursued for 15 min, after check of coronary flow. Then the heart is arrested with 50 mL Celsior + molecule to rest at 4°C under a pressure of 60cm H₂0. The heart is then removed and stored 5 hours at 4°C in plastic container filled with the same solution and surrounded with crushed ice.

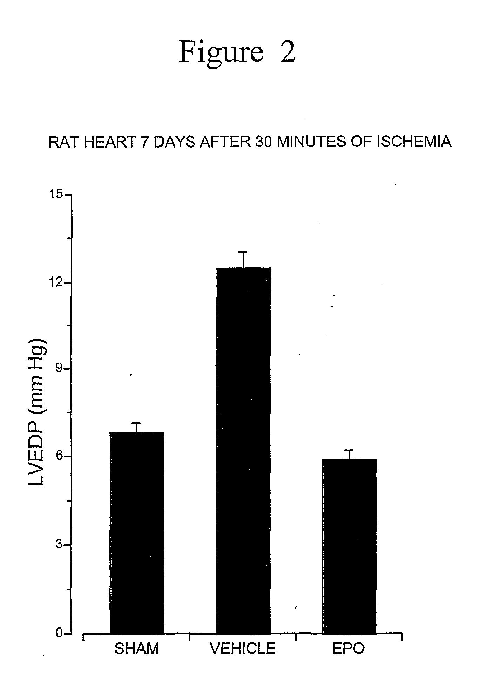

On completion of storage, the heart is transferred to a Langendorff apparatus. The balloon catheter is re-inserted into the left ventricle and re-inflated to the same volume as during preischemic period. The heart is re-perfused for at least 2 hours at 37°C. The re-perfusion pressure is set at 50cm H₂0 for 15min of re-flow and then back to 100cm H₂0 for the 2 next hours. Pacing (320 beats per minute) is re-instituted. Isovolumetric measurements of contractile indexes and diastolic pressure are taken in triplicate at 25, 45, 60, 120 min of reperfusion. At this time point pressure volume curves are performed and coronary effluent during the 45mn reperfusion collected to measure creatine kinase leakage. The two treatment groups are compared using an unpaired t-test, and a linear regression using the end-diastolic pressure data is used to design compliance curves. As shown in Figure 2, significant improvement of left ventricular pressure developed occurs after treatment with erythropoietin, as well as improved volume-pressure curve, decrease of left diastolic ventricular pressure and decrease of creatine kinase leakage.

### Example 3

### ERYTHROPOIETIN PROTECTS MYOCARDIUM FROM ISCHEMIC INJURY.

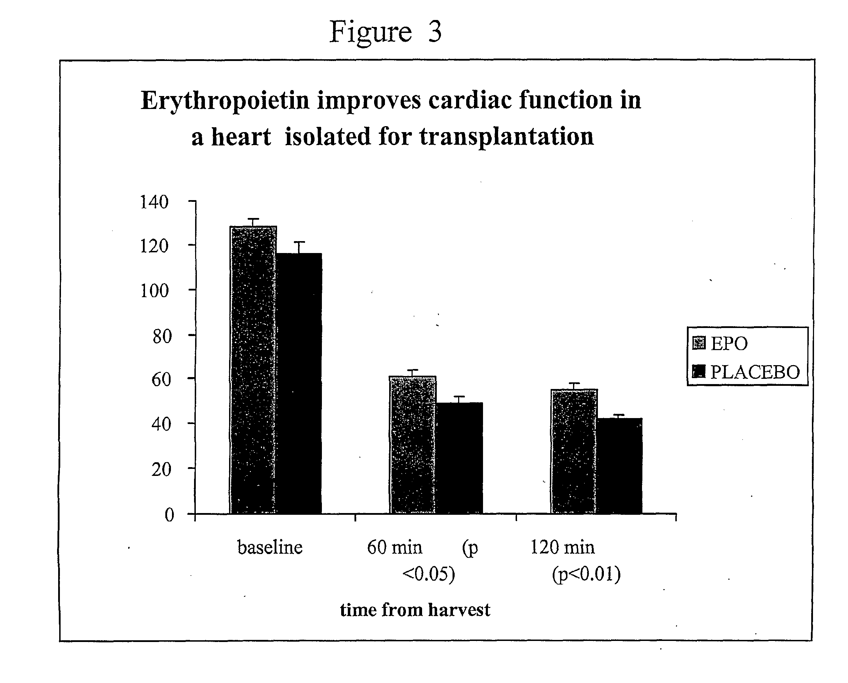

Adult male rats given recombinant human erythropoietin (5000 U/kg body weight) 24 hrs previously are anesthetized and prepared for coronary artery occlusion. An additional dose of erythropoietin is given at the start of the procedure and the left main coronary artery occluded for 30 minutes and then released. The same dose of erythropoietin is given daily for one week after treatment. The animals are then studied for cardiac function. As **Figure 3** illustrates, animals receiving a sham injection (saline) demonstrated a large increase in the left end diastolic pressure, indicative of a dilated, stiff heart secondary to myocardial infarction. In contradistinction, animals receiving erythropoietin suffered no decrement in cardiac function, compared to sham operated controls (difference significant at the p < 0.01 level).

### Example 4

### ERYTHROPOIETIN MOLECULES

Native erythropoietin may be modified to tailor its activities for a specific tissue or tissues. Several non-limiting strategies that may be carried out to achieve this desired tissue specificity include modifications that remove or modify the glycosylation moieties, of which erythropoietin has three N-linked and one O-linked. Such variants of glycosylated erythropoietin can be produced in a number of ways. For example, the sialic acids which terminate the end of the sugar chains can be removed by specific sialidases depending on the chemical linkage connecting the sialic acid to the sugar chain. Alternatively, the glycosylated structure can be dismantled in different ways by using other enzymes that cleave at specific linkages. To validate these principles, recombinant human erythropoietin was desialized using Sialidase A (Prozyme Inc.) according to the manufacturer's protocol. Successful chemical modification was confirmed by running the reaction product on an SDS polyacrylamide gel and staining the resultant bands which showed that the chemically-modified erythropoietin possessed an apparent molecular weight of ∼ 31 kD as expected, compared to unmodified erythropoietin which was ∼34 kD and by measuring the sialic acid residues remaining by chemical means to be < 0.1 mole/ mole of erythropoietin.

In another modification wherein the amino acid residues of erythropoietin are modified, arginine residues were modified by using phenylglyoxal according to the protocol of Takahashi (1977, J. Biochem. 81:395-402) carried out for variable lengths of time ranging from 0.5 to 3 hrs at room temperature. The reaction was terminated by dialyzing the reaction mixture against water. Use of such modified forms of erythropoietin is fully embraced herein.

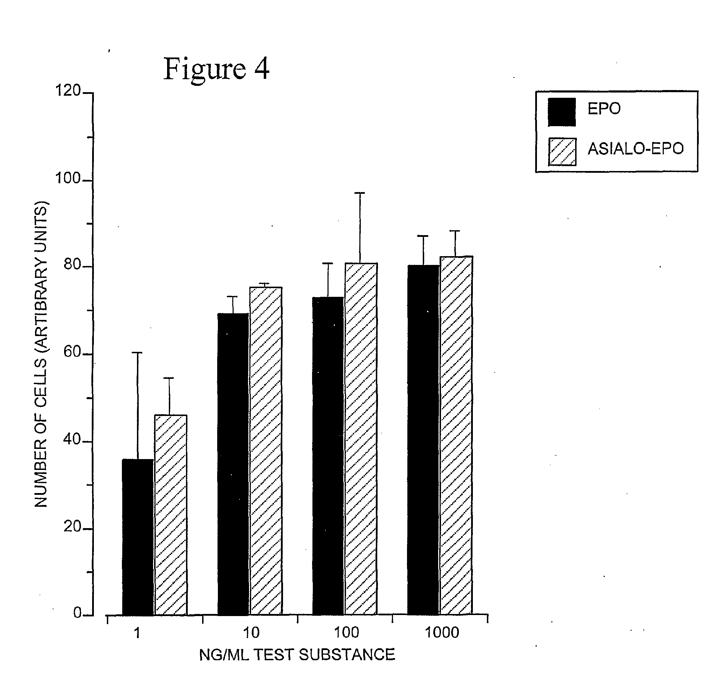

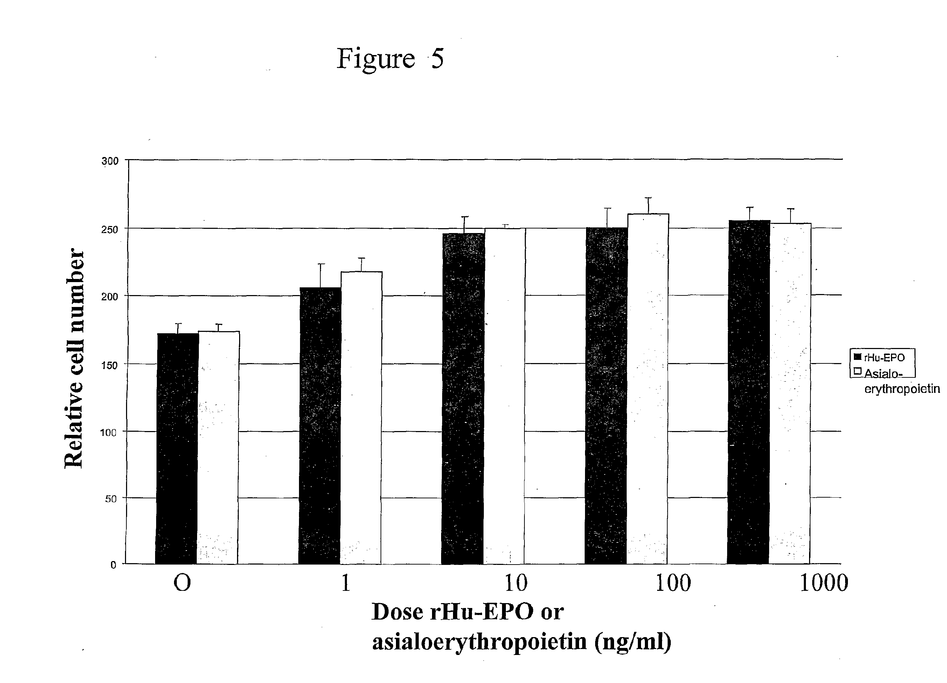

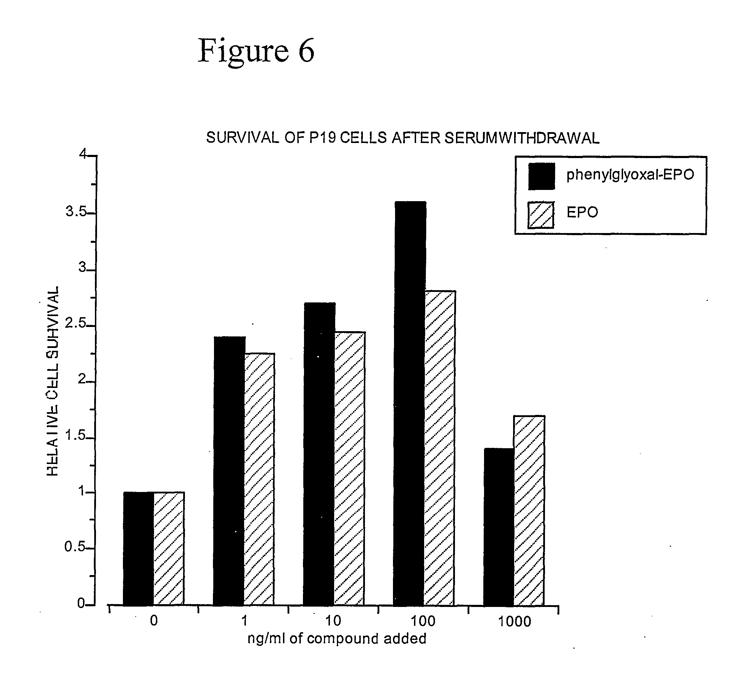

Asialoerythropoietin and phenylglyoxalerythropoietin were as effective as native erythropoietin for neural cells *in vitro* as shown in **Figures 4-6****.** *In-vitro* testing was carried out using neural-like embryonal carcinoma cells (P19) that undergo apoptosis upon the withdrawal of serum. Twenty-four hours before the removal of serum, 1-1000 ng/ml of erythropoietin or a modified erythropoietin was added to the cultures. The following day the medium was removed, the cells washed with fresh, non-serum containing medium, and medium containing the test substance (no serum) added back to the cultures for and additional 48 hours. To determine the number of viable cells, a tetrazolium reduction assay was performed (CellTiter 96; Promega, Inc.). As **Figure 4-5** illustrate, asialoerythropoietin appears to be of equal potency to erythropoietin itself in preventing cell death. The phenylglyoxal-modified erythropoietin was tested using the neural-like P19 cell assay described above. As **Figure 6** illustrates, this chemically-modified erythropoietin fully retains its neuroprotective effects.

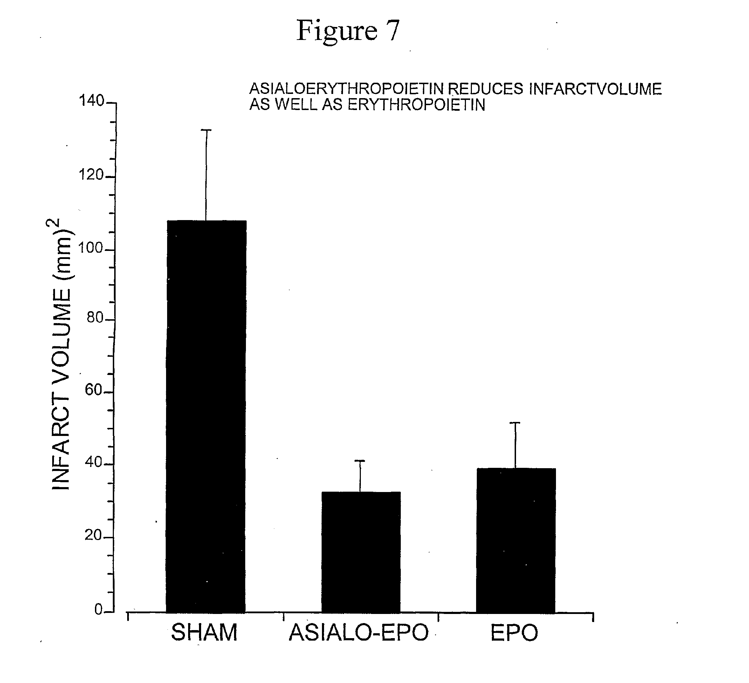

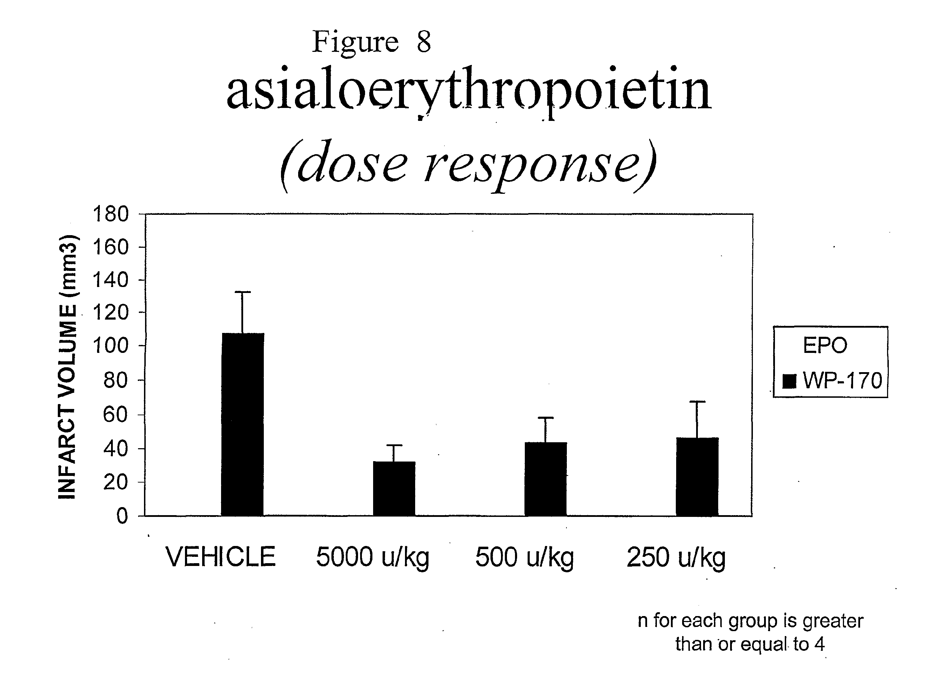

Retention of neuroprotective activity *in vivo* was confirmed using a rat focal ischemia model in which a reversible lesion in the territory of the middle cerebral artery is performed as described previously (Brines et al., 2000, Proc. Nat. Acad. Sci. U.S.A. 97:10526-31). Adult male Sprague-Dawley rats were administered asialoerythropoietin or erythropoietin (5000 U/kgBW intraperitoneally) or vehicle at the onset of the arterial occlusion. Twenty-four hours later, the animals were sacrificed and their brains removed for study. Serial sections were cut and stained with tetrazolium salts to identify living regions of the brain. As shown in **Figure 7****,** asialoerythropoietin was as effective as native erythropoietin in providing neuroprotection from 1 hour of ischemia. **Figure 8** shows the results of another focal ischemia model in which a comparative dose response was performed with erythropoietin and asialoerythropoietin. At the lowest dose of 250 U/kg, asialoerythropoietin afforded protection whereas unmodified erythropoietin did not.

### Example 5

### MODIFICATION OF PRIMARY STRUCTURE OF ERYTHROPOIETIN AND EFFECTIVENESS AT NEURONAL PROTECTION

A number of mutant erythropoietin molecules have been described which do not bind to the erythrocyte erythropoietin receptor and thus do not support erythropoiesis *in vivo* or *in vitro.* Some of these molecules will nevertheless mimic the actions of erythropoietin itself in other tissues or organs. For example, a 17-mer containing the amino-acid sequence of 31-47 of native erythropoietin is inactive for erythropoiesis but fully active for neural cells *in vitro* (Campana & O'Brien, 1998: Int. J. Mol. Med. 1:235-41).

Derivative erythropoietins desirable for the uses described herein may be generated by guanidination, amidination, trinitrophenylation, acetylation, succinylation, nitration, or modification of arginine residues or carboxyl groups, among other procedures as mentioned herein above, to produce erythropoietins which maintain their activities for specific organs and tissues but not for others, such as erythrocytes. When erythropoietin is subjected to the above reactions, it has been found that in general the resultant molecule lacks both *in-vivo* and *in-vitro* erythropoietic activity (e.g., Satake et al; 1990, Biochim. Biophys. Acta 1038:125-9). Some examples of the preparation of modified erythropoietins are described below.

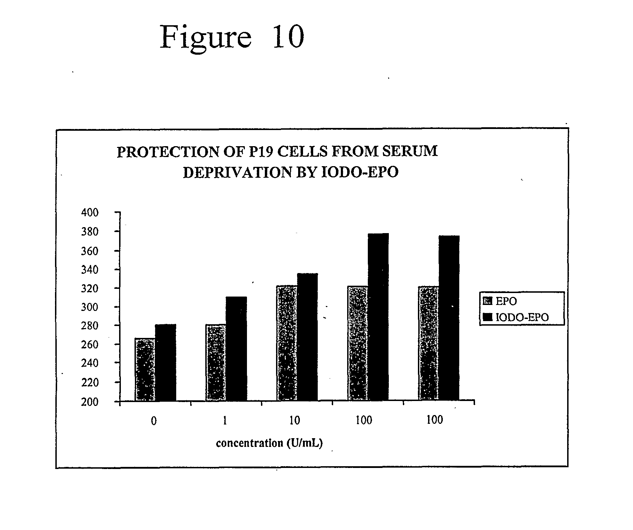

Biotinylation at free amino groups of erythropoietin. 0.2 mg D-biotinoyl-e-aminocaproic acid-N-hydroxysuccinimide ester (Boehringer Mannheim #1418165) was dissolved in 100 ul DMSO. This solution was combined with 400 ul PBS containing approximately 0.2 mg erythropoietin in a foil covered tube. After incubation for 4 hours at room temperature, the unreacted biotin was separated by gel filtration on a Centricon 10 column. As shown by Figure 10, this biotinylated erythropoietin protects p19 cells from serum withdrawal.

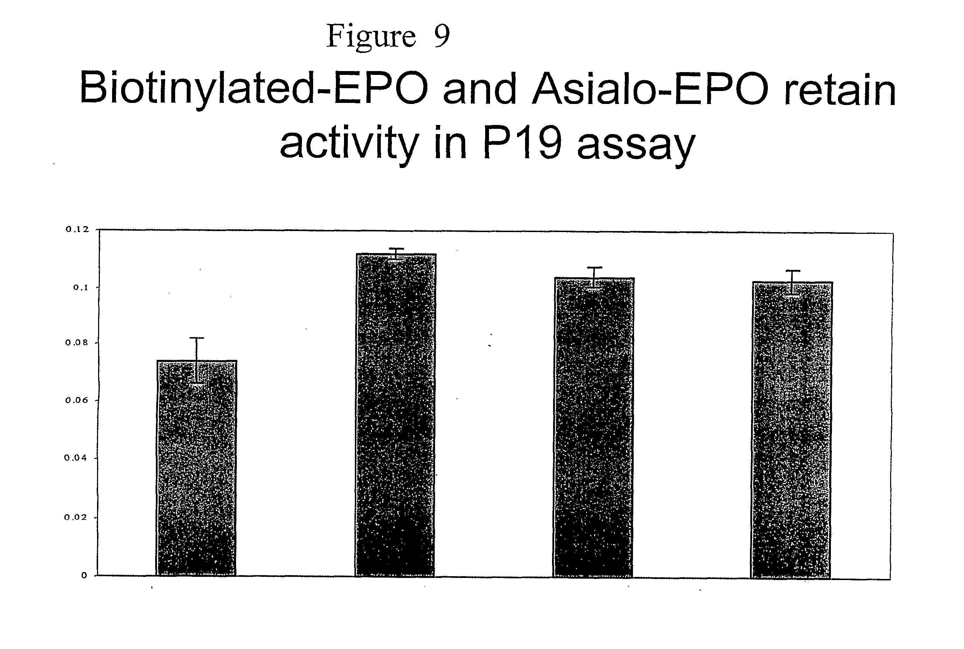

In "Biotinylated recombinant human erythropoietins: Bioactivity and Utility as a receptor ligand" by Wojchowski et al. Blood, 1989, 74(3):952-8, the authors use three different methods of biotinylating erythropoietin. Biotin is added to (1) the sialic acid moieties (2) carboxylate groups (3) amino groups. The authors use a mouse spleen cell proliferation assay to demonstrate that (1) the addition of biotin to the sialic acid moieties does not inactivate the biological activity of erythropoietin (2) the addition of biotin to carboxylate groups led to substantial biological inactivation of erythropoietin (3) the addition of biotin to amino groups resulted in complete biological inactivation of erythropoietin. These methods and modifications are fully embraced herein. **Figure 9** shows the activity of biotinylated erythropoietin and asialoerythropoietin in the serum-starved P19 assay.

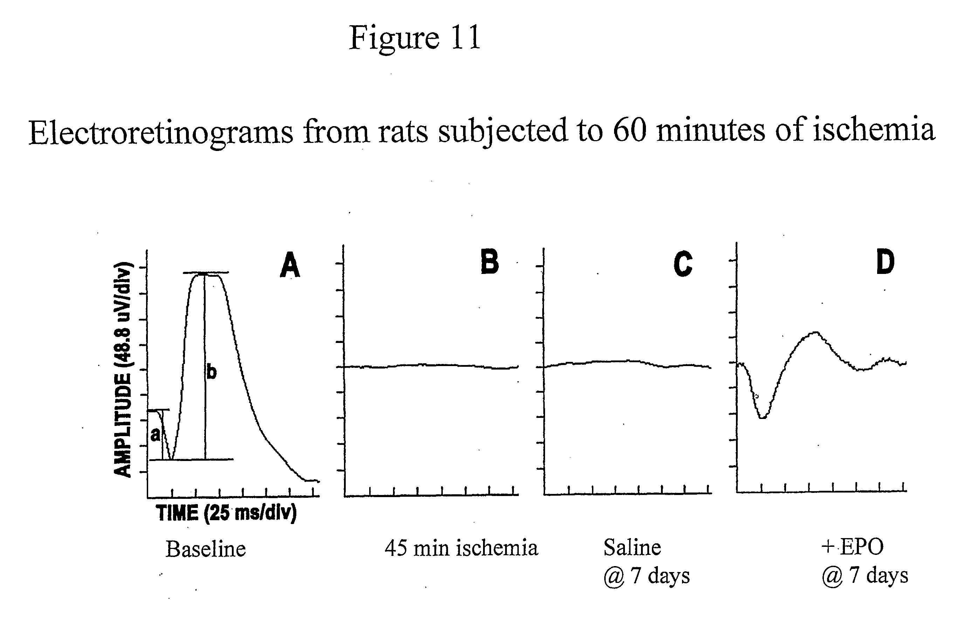

Iodination of erythropoietin. Method 1 - Iodo Beads. One Iodo Bead (Pierce, Rockford, Il) was incubated in 100 ul PBS (20mM sodium phosphate, 0.15M NaCl, pH7.5) containing 1 mCi free Na¹²⁵I for 5 minutes. 100 ug erythropoietin in 100 ul PBS was then added to the mixture. After a ten minute incubation period at room temperature, the reaction was stopped by removing the 200ul solution from the reaction vessel (leaving the iodo bead behind). The excess iodine was removed by gel filtration on a Centricon 10 column. As shown in Figure 11, iodo-erythropoietin produced in this manner is efficacious in protecting P19 cells from serum withdrawal.

Method 2 - Chloramine T. 100 ug erythropoietin in 100 ul PBS was added to 500 uCi Na¹²⁵I were mixed together in an eppendorf tube. 25 ul chloramines T (2 mg/ml) was then added and the mixture was incubated for 1 minute at room temperature. 50 ul of Chloramine T stop buffer (2.4 mg/ml sodium metabisulfite, 10 mg/ml tyrosine, 10% glycerol, 0.1% xylene in PBS was then added. The iodotyrosine and iodinated erythropoietin were then separated by gel filtration on a Centricon 10 column.

Lysine modifications: Carbamylation: erythropoietin (100 ug) was modified with potassium cyanate as described in Plapp et al ("Activity of bovine pancreatic deoxyribonuclease A with modified amino groups" 1971, J. Biol. Chem. 246, 939-845).

Trinitrophenylation: erythropoietin (100 ug) was modified with 2,4,6-trinitrobenzenesulfonate as described in Plapp et al ("Activity of bovine pancreatic deoxyribonuclease A with modified amino groups" 1971, J. Biol. Chem. 246, 939-845)

Acetylation: erythropoietin (100 ug) was incubated in 0.3M phosphate buffer (pH7.2) containing an equal amount of acetic anhydride at 0 C for 1 hour. The reaction was stopped by dialysis against distilled water.

Succinylation: erythropoietin (100 ug) in 0.5 M NaHCO3 (pH 8.0) was incubated with a 15 molar excess of succinic anhydride at 15 C for 1 hour. The reaction was stopped by dialysis against distilled water.

Arginine modifications: erythropoietin was modified with 2,3 butanedione as described in Riordan ("Functional arginyl residues in carboxypeptidase A. Modification with butanedione" Riordan JF, Biochemistry 1973, 12(20): 3915-3923).

Erythropoietin was modified with cylcohexanone as in Patthy et al ("Identification of functional arginine residues in ribonuclease A and lysozyme" Patthy, L, Smith EL, J. Biol. Chem 1975 250(2): 565-9).

Erythropoietin was modified with phenylglyoxal as described in Werber et al. ("Proceedings: Carboxypeptidase B: modification of functional arginyl residues" Werber, MM, Sokolovsky M Isr J Med Sci 1975 11(11): 1169-70).

Tyrosine modifications: erythropoietin (100 ug) was incubated with tetranitromethane as previously described in Nestler et al "Stimulation of rat ovarian cell steroidogenesis by high density lipoproteins modified with tetranitromethane" Nestler JE, Chacko GK, Strauss JF 3rd. J Biol Chem 1985 Jun 25;260(12):7316-21).

Glutamic acid (and aspartic acid) modifications: In order to modify carboxyl groups, erythropoietin (100 ug) was incubated with 0.02 M EDC in 1M glycinamide at pH 4.5 at room temperature for 60 minutes as described in Carraway et al "Carboxyl group modification in chymotrypsin and chymotrypsinogen." Carraway KL, Spoerl P, Koshland DE Jr. J Mol Biol 1969 May 28;42(1):133-7.

Tryptophan residue modifications: erythropoietin (100 ug) was incubated with 20 uM n-bromosuccinimide in 20 mM potassium phosphate buffer (pH 6.5) at room temperature as described in Ali et al., J Biol Chem. 1995 Mar 3;270(9):4570-4. The number of oxidized tryptophan residues was determined by the method described in Korotchkina (Korotchkina, LG et al Protein Expr Purif. 1995 Feb;6(1):79-90).

Removal of amino groups: In order to remove amino groups of erythropoietin (100 ug) was incubated with in PBS (pH 7.4) containing 20mM ninhydrin (Pierce Chemical, Rockford, Il), at 37 C for two hours as in Kokkini et al (Kokkini, G., et al "Modification of hemoglobin by ninhydrin" Blood, Vol. 556, No 4 1980: 701-705). Reduction of the resulting aldehyde was accomplished by reacting the product with Sodium borohydride or lithium aluminum hydride. Specifically, erythropoietin (100 ug) was incubated with 0.1M sodium borohydride in PBS for 30 minutes at room temperature. The reduction was terminated by cooling the samples on ice for 10 minutes and dialyzing it against PBS, three times, overnight. (Kokkini, G., Blood, Vol. 556, No 4 1980: 701-705). Reduction using lithium aluminum hydride was accomplished by incubating erythropoietin (100 ug) with 0.1M lithium aluminum hydride in PBS for 30 minutes at room temperature. The reduction was terminated by cooling the samples on ice for 10 minutes and dialyzing it against PBS, three times, overnight.

Disulfide reduction and stabilization: erythropoietin (100 ug) was incubated with 500 mM DTT for 15 minutes at 60 C. 20 mM iodoacetamide in water was then added to the mixture and incubated for 25 minutes, at room temperature in the dark.

Limited proteolysis: Erythropoietin can be subjected to a limited chemical proteolysis that targets specific residues. Erythropoietin was reacted with 2-(2-nitrophenylsulfenyl)-3-methyl-3'-bromoindolenine which cleaves specifically after tryptophan residues in a 50 times excess in 50% acetic acid for 48 hours in the dark at room temperature in tubes capped under nitrogen pressure. The reaction was terminated by quenching with tryptophan and desalting.

### Example 6

### PROTECTION OF RETINAL ISCHEMIA BY PERIPHERALLY-ADMINISTERED ERYTHROPOIETIN.

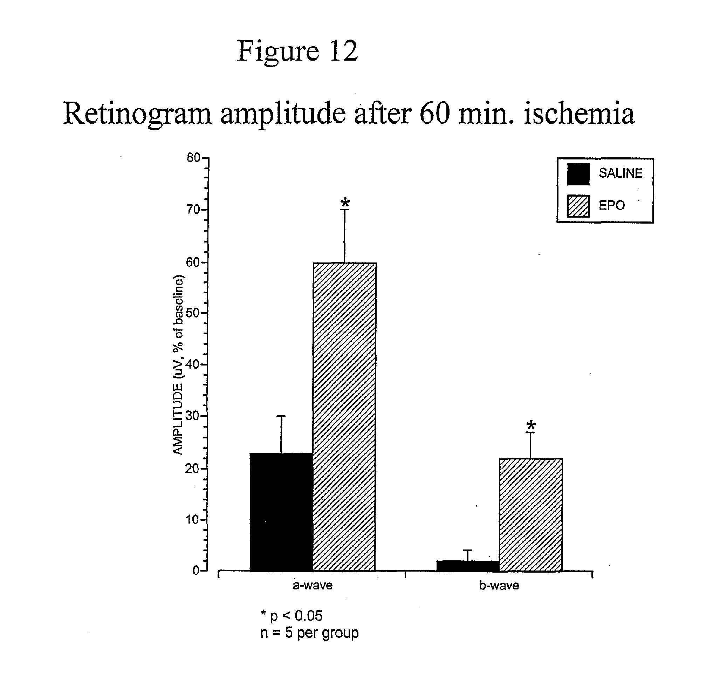

Retinal cells are very sensitive to ischemia such that many will die after 30 minutes of ischemic stress. Further, subacute or chronic ischemia underlies the deterioration of vision which accompanies a number of common human diseases, such as diabetes mellitus, glaucoma, and macular degeneration. At the present time there are no effective therapies to protect cells from ischemia. A tight endothelial barrier exists between the blood and the retina that excludes most large molecules. To test whether peripherally-administered erythropoietin will protect cells sensitive to ischemia, an acute, reversible glaucoma rat model was utilized as described by Rosenbaum et al. (1997; Vis. Res. 37:3443-51). Specifically, saline was injected into the anterior chamber of the eye of adult male rats to a pressure above systemic arterial pressure and maintained for 60 minutes. Animals were administered saline or 5000 U erythropoietin/kg body weight intraperitoneally 24 hours before the induction of ischemia, and continued as a daily dose for 3 additional days. Electroretinography was performed on dark-adapted rats 1 week after treatment. Figure 11-12 illustrate that the administration of erythropoietin is associated with good preservation of the electroretinogram (ERG) (Panel D), in contrast to animals treated with saline alone (Panel C), for which very little function remained. Figure 11 compares the electroretinogram a- and b-wave amplitudes for the erythropoietin-treated and saline-treated groups, and shows significant protection afforded by erythropoietin.

### EXAMPLE 7

### RESTORATIVE EFFECTS OF ERYTHROPOIETIN ON DIMINSHED COGNITIVE FUNCTION ARISING FROM BRAIN INJURY

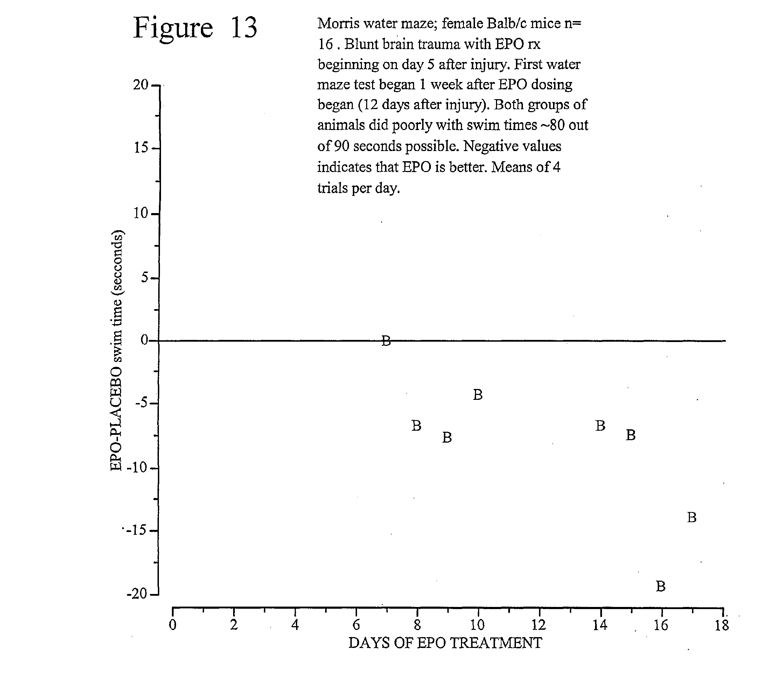

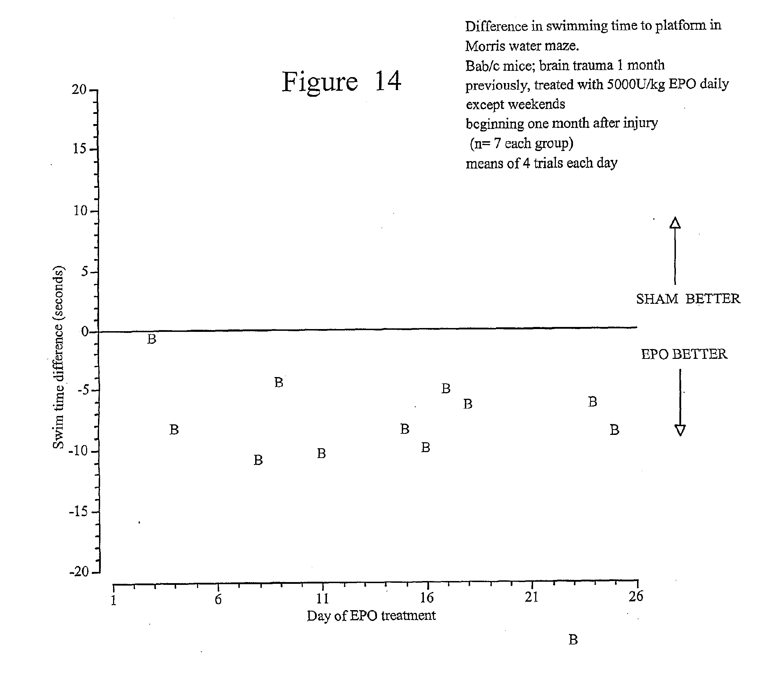

In a study to demonstrate the ability of erythropoietin to restore diminished cognitive function in mice after receiving brain trauma, female Balb/c mice were subject to blunt brain trauma as described in Brines et al. PNAS 2000, 97; 10295-10672 and five days later, daily erythropoietin administration of 5000 U/kg-bw intraperitoneally was begun. Twelve days after injury, animals were tested for cognitive function in the Morris water maze, with four trials per day. While both treated and untreated animals performed poorly in the test (with swim times of about 80 seconds out of a possible 90 seconds), Figure 13 shows that the erythropoietin-treated animals performed better (in this presentation, a negative value is better). Even if the initiation of erythropoietin treatment is delayed until 30 days after trauma (Figure 14), restoration of cognitive function is also seen.

### Example 8

### KAINATE MODEL

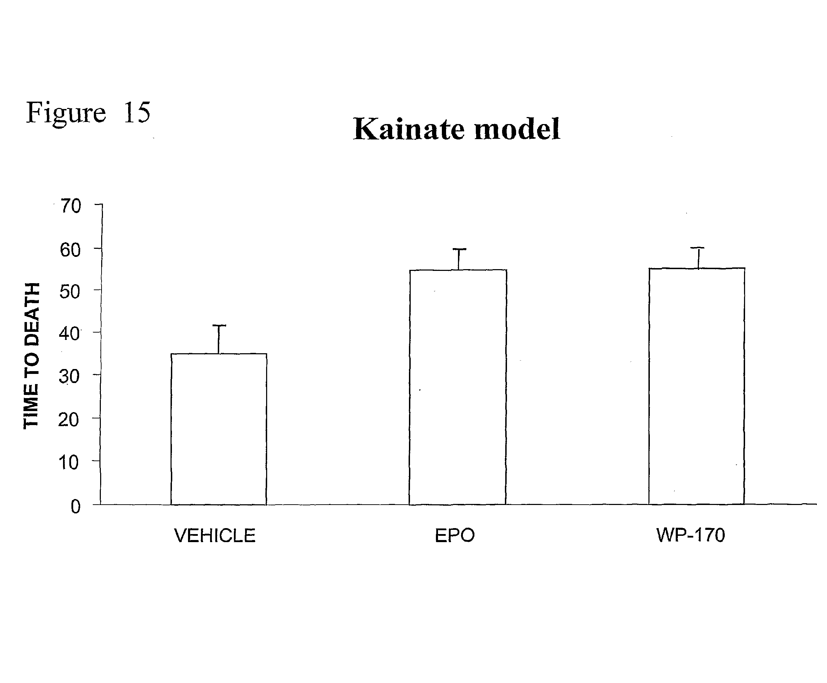

In the kainate neurotoxicity model, asialoerythropoietin was administered according to the protocol of Brines et al. Proc. Nat. Acad. Sci. U.S.A. 2000, 97; 10295-10672 at a dose of 5000U/kg-bw given intraperitoneally 24 hours before the administration of 25 mg/kg kainate is shown to be as effective as erythropoietin, as shown by time to death **(****Figure 15****).**

The invention is not to be limited in scope by the specific embodiments described which are intended as single illustrations of individual aspects of the invention. Indeed various modifications of the invention, in addition to those shown and described herein will become apparent to those skilled in the art from the foregoing description and accompanying drawings. Such modifications are intended to fall within the scope of the appended claims.

## Claims

1. Pharmaceutical composition comprising a modified, non-erythropoietic erythropoietin and a pharmaceutically-acceptable carrier, for use in protecting, enhancing or restoring the function or viability of an erythropoietin-responsive neuronal mammalian tissue comprising erythropoietin-responsive cells, wherein said modified erythropoietin has at least one of the following modifications:
- 0, 1, 2, 3, 4, 5, 6, 7, 8, 9, 10, 11, 12, or 13 sialic acid moieties; or
- one or more carbamylated lysine residues and/or a carbamylation of the N-terminal amino group.

2. Pharmaceutical composition for use of claim 1 wherein a lysine residue of said modified erythropoietin is carbamylated.

3. Pharmaceutical composition for use of claim 1 wherein said modified erythropoietin is carbamylated asialoerythropoietin.

4. Pharmaceutical composition for use of claim 1 wherein said modified erythropoietin is biotinylated asialoerythropoietin.

5. Pharmaceutical composition for use of claim 1 wherein said modified erythropoietin is succinylated asialoerythropoietin.

6. Pharmaceutical composition for use of claim 1 wherein said modified erythropoietin is asialoerythropoietin.

7. Pharmaceutical composition for use of claim 6 wherein said asialoerythropoietin is human asialoerythropoietin.

8. Pharmaceutical composition for use of any of the claims 1 to 6 wherein said erythropoietin is human erythropoietin.

9. Pharmaceutical composition for use of claim 1 wherein the modified erythropoietin has one or more carbamylated lysine residues and/or a carbamylation of the N-terminal amino group and wherein said erythropoietin is human erythropoietin.

10. Pharmaceutical composition for use of any of claims 1 to 9 wherein said modified erythropoietin is expressed in an insect or plant cell.

11. Pharmaceutical composition for use of any of claims 1 to 10 wherein said modified erythropoietin is capable of the treatment or prevention of a disease or disorder of the central nervous system or peripheral nervous system.

12. Pharmaceutical composition for use of any of claims 1 to 11 wherein said modified erythropoietin is capable of the treatment or prevention of a disease or disorder of the brain, eye or retina in a mammal.

13. Pharmaceutical composition for use of claims 1 to 10 wherein said modified erythropoietin is effective for the restoration or enhancement of cognitive function in a mammal having, having had, or at risk for cognitive dysfunction.

14. Pharmaceutical composition for use of claim 13 wherein said cognitive dysfunction is caused by brain trauma.

15. Pharmaceutical composition for use of claim 13 wherein said enhancement of cognitive function is enhancement of learning.

16. Pharmaceutical composition for use of any one of claims 1 to 12 wherein the mammal, has, has had, or is at risk for, or is undergoing multiple sclerosis, stroke, central nervous system injury, neuronal loss, ischemia, neurodegenerative disease or disorder, brain injury or spinal cord injury, post-operative treatment for embolic or ischemic injury, diabetic neuropathy, eye tissue damage, macular degeneration, diabetic retinopathy, glaucoma, retinal ischemia, retinal trauma, retinitis pigmentosa, optic nerve damage, retinal detachment, arteriosclerotic retinopathy, hypertensive retinopathy, retinal artery blockage and/or retinal vein blockage.

17. Pharmaceutical composition for use of claim 16 wherein the neurodegenerative disease or disorder is selected from Alzheimer's disease, Parkinson's disease, Huntington's disease, Tourette's syndrome, Leigh disease, Guillian Barre, amyotrophic lateral sclerosis and Friedreich's ataxia.

## Patentansprüche

1. Pharmazeutische Zusammensetzung, umfassend ein modifiziertes, nicht-erythropoietisches Erythropoietin und einen pharmazeutisch annehmbaren Träger zur Verwendung zum Schützen, Verbessern oder Wiederherstellen der Funktion oder der Lebensfähigkeit eines auf Erythropoietin ansprechenden neuronalen Säugergewebes, umfassend auf Erythropoietin ansprechende Zellen, wobei das modifizierte Erythropoietin zumindest eine der nachstehenden Modifikationen aufweist:
- 0, 1, 2, 3, 4, 5, 6, 7, 8, 9, 10, 11, 12 oder 13 Sialinsäuregruppen; oder
- einen oder mehrere carbamylierte Lysinrest(e) und/oder eine Carbamylierung der N-terminalen Aminogruppe.

2. Pharmazeutische Zusammensetzung zur Verwendung gemäss Anspruch 1, wobei ein Lysinrest des modifizierten Erythropoietins carbamyliert ist.

3. Pharmazeutische Zusammensetzung zur Verwendung gemäss Anspruch 1, wobei das modifizierte Erythropoietin carbamyliertes Asialoerythropoietin ist.

4. Pharmazeutische Zusammensetzung zur Verwendung gemäss Anspruch 1, wobei das modifizierte Erythropoietin biotinyliertes Asialoerythropoietin ist.

5. Pharmazeutische Zusammensetzung zur Verwendung gemäss Anspruch 1, wobei das modifizierte Erythropoietin succinyliertes Asialoerythropoietin ist.

6. Pharmazeutische Zusammensetzung zur Verwendung gemäss Anspruch 1, wobei das modifizierte Erythropoietin Asialoerythropoietin ist.

7. Pharmazeutische Zusammensetzung zur Verwendung gemäss Anspruch 6, wobei das Asialoerythropoietin menschliches Asialoerythropoietin ist.

8. Pharmazeutische Zusammensetzung zur Verwendung gemäss einem der Ansprüche 1 bis 6, wobei das Erythropoietin menschliches Erythropoietin ist.

9. Pharmazeutische Zusammensetzung zur Verwendung gemäss Anspruch 1, wobei das modifizierte Erythropoietin einen oder mehrere carbamylierte Lysinrest(e) und/oder eine Carbamylierung der N-terminalen Aminogruppe aufweist und wobei das Erythropoietin menschliches Erythropoietin ist.

10. Pharmazeutische Zusammensetzung zur Verwendung gemäss einem der Ansprüche 1 bis 9, wobei das modifizierte Erythropoietin in einer Insekten- oder Pflanzenzelle exprimiert wird.

11. Pharmazeutische Zusammensetzung zur Verwendung gemäss einem der Ansprüche 1 bis 10, wobei das modifizierte Erythropoietin zur Behandlung oder Prävention einer Erkrankung oder Störung des zentralen Nervensystems oder des peripheren Nervensystems fähig ist.

12. Pharmazeutische Zusammensetzung zur Verwendung gemäss einem der Ansprüche 1 bis 11, wobei das modifizierte Erythropoietin zur Behandlung oder Prävention einer Erkrankung oder Störung des Gehirns, des Auges oder der Netzhaut in einem Säuger fähig ist.

13. Pharmazeutische Zusammensetzung zur Verwendung gemäss den Ansprüchen 1 bis 10, wobei das modifizierte Erythropoietin zur Wiederherstellung oder Verbesserung der kognitiven Funktion bei einem Säuger wirksam ist, der eine kognitive Dysfunktion hat, gehabt hat oder davon bedroht ist.

14. Pharmazeutische Zusammensetzung zur Verwendung gemäss Anspruch 13, wobei die kognitive Dysfunktion durch ein Hirntrauma verursacht ist.

15. Pharmazeutische Zusammensetzung zur Verwendung gemäss Anspruch 13, wobei die Verbesserung einer kognitiven Funktion eine Verbesserung des Lernens ist.

16. Pharmazeutische Zusammensetzung zur Verbesserung gemäss irgendeinem der Ansprüche 1 bis 12, wobei der Säuger, Multiple Sklerose, einen Schlaganfall, eine Verletzung des zentralen Nervensystems, Neuronenverlust, Ischämie, eine neurodegenerativer Erkrankung oder Störung, eine Hirnverletzung oder Rückenmarksverletzung, eine post-operative Behandlung einer embolischen oder ischämischen Verletzung, diabetische Neuropathie, Augengewebeschäden, Makuladegeneration, diabetische Retinopathie, Glaukom, retinale Ischämie, Netzhauttrauma, Retinitis pigmentosa, eine Schädigung des Sehnervs, Netzhautablösung, arteriosklerotische Retinopathie, hypertensive Retinopathie, eine Blockade der Netzhautarterie und/oder eine Venenblockade hat, gehabt hat, davon bedroht ist oder erfährt.

17. Pharmazeutische Zusammensetzung zur Verwendung gemäss Anspruch 16, wobei die neurodegenerative Erkrankung oder Störung aus Alzheimer-Krankheit, Parkinson-Krankheit, Huntington-Krankheit, Tourette-Syndrome, Leigh-Krankheit, Guillian Barre, amyotropher Lateralsklerose und Friedreich-Ataxie ausgewählt ist.

## Revendications

1. Composition pharmaceutique comprenant une érythropoïétine modifiée, non érythropoïétique, et un support pharmaceutiquement acceptable, destinée à être utilisée dans la protection, l'amélioration ou le rétablissement de la fonction ou de la viabilité d'un tissu neuronal de mammifère sensible à l'érythropoïétine comprenant des cellules sensibles à l'érythropoïétine, dans laquelle ladite érythropoïétine modifiée présente au moins l'une des modifications suivantes :
- 0, 1, 2, 3, 4, 5, 6, 7, 8, 9, 10, 11, 12, ou 13 fractions d'acide sialique ; ou
- un ou plusieurs résidus lysine carbamylés et/ou une carbamylation du groupe amino N-terminal.

2. Composition pharmaceutique destinée à être utilisée selon la revendication 1, dans laquelle un résidu lysine de ladite érythropoïétine modifiée est carbamylé.

3. Composition pharmaceutique destinée à être utilisée selon la revendication 1, dans laquelle ladite érythropoïétine modifiée est une asialoérythropoïétine carbamylée.

4. Composition pharmaceutique destinée à être utilisée selon la revendication 1, dans laquelle l'érythropoïétine modifiée est une asialoérythropoïétine biotinylée.

5. Composition pharmaceutique destinée à être utilisée selon la revendication 1, dans laquelle l'érythropoïétine modifiée est une asialoérythropoïétine succinylée.

6. Composition pharmaceutique destinée à être utilisée selon la revendication 1, dans laquelle ladite érythropoïétine modifiée est une asialoérythropoïétine.

7. Composition pharmaceutique destinée à être utilisée selon la revendication 6, dans laquelle ladite asialoérythropoïétine est l'asialoérythropoïétine humaine.

8. Composition pharmaceutique destinée à être utilisée selon l'une quelconque des revendications 1 à 6, dans laquelle l'érythropoïétine est l'érythropoïétine humaine.

9. Composition pharmaceutique destinée à être utilisée selon la revendication 1, dans laquelle l'érythropoïétine modifiée présente un ou plusieurs résidus lysine carbamylés et/ou une carbamylation du groupe amino N-terminal et dans laquelle ladite érythropoïétine est l'érythropoïétine humaine.

10. Composition pharmaceutique destinée à être utilisée selon l'une quelconque des revendications 1 à 9, dans laquelle ladite érythropoïétine modifiée est exprimée dans une cellule d'insecte ou de plante.

11. Composition pharmaceutique destinée à être utilisée selon l'une quelconque des revendications 1 à 10, dans laquelle ladite érythropoïétine modifiée est apte au traitement ou à la prévention d'une maladie ou d'un trouble du système nerveux central ou du système nerveux périphérique.

12. Composition pharmaceutique destinée à être utilisée selon l'une quelconque des revendications 1 à 11, dans laquelle ladite érythropoïétine modifiée est apte au traitement ou à la prévention d'une maladie ou d'un trouble du cerveau, de l'oeil ou de la rétine chez un mammifère.

13. Composition pharmaceutique destinée à être utilisée selon les revendications 1 à 10, dans laquelle ladite érythropoïétine modifiée est efficace pour le rétablissement ou l'amélioration de la fonction cognitive chez un mammifère présentant, ayant présenté, ou à risque de dysfonctionnement cognitif.

14. Composition pharmaceutique destinée à être utilisée selon la revendication 13, dans laquelle ledit dysfonctionnement cognitif est provoqué par un traumatisme cérébral.

15. Composition pharmaceutique destinée à être utilisée selon la revendication 13, dans laquelle ladite amélioration de la fonction cognitive est une amélioration de l'apprentissage.

16. Composition pharmaceutique destinée à être utilisée selon l'une quelconque des revendications 1 à 12, dans laquelle le mammifère, présente, a présenté, ou est à risque de, ou subit une sclérose en plaques, un accident vasculaire cérébral, une lésion du système nerveux central, une perte neuronale, une ischémie, une maladie ou un trouble neurodégénératif, une lésion cérébrale ou une lésion de la moelle épinière, un traitement post-opératoire d'une lésion embolique ou ischémique, une neuropathie diabétique, des lésions du tissu oculaire, une dégénérescence maculaire, une rétinopathie diabétique, un glaucome, une ischémie rétinienne, un traumatisme rétinien, une rétinite pigmentaire, des dommages du nerf optique, un décollement de la rétine, une rétinopathie liée à l'artériosclérose, une rétinopathie hypertensive, un blocage de l'artère rétinienne et/ou un blocage de la veine de la rétine.

17. Composition pharmaceutique destinée à être utilisée selon la revendication 16, dans laquelle la maladie ou le trouble neurodégénératif est choisi parmi la maladie d'Alzheimer, la maladie de Parkinson, la maladie de Huntington, le syndrome de Tourette, la maladie de Leigh, le syndrome de Guillain-Barré, la sclérose latérale amyotrophique et l'ataxie de Friedreich.
